# EUROPEAN PATENT SPECIFICATION

(11) **EP 2 991 963 B1**
(45) Date of publication and mention of the grant of the patent: **07.07.2021**
(21) Application number: 14719748.7
(22) Date of filing: 25.04.2014
(51) Int. Cl.: C07C 59/70, C07D 207/337, C07D 209/42, C07D 235/28, C07D 307/68, C07D 317/60, C07D 471/04, C07D 493/04, A61K 31/194, A61K 31/341, A61K 31/35, A61K 31/36, A61K 31/40, A61K 31/404, A61K 31/4184

(54) **INHIBITORS OF NHR2 AND/OR RUNX1/ETO-TETRAMERIZATION**
INHIBITOREN DER NHR2- UND/ODER RUNX1/ETO-TETRAMERISIERUNG
INHIBITEURS DE LA TÉTRAMÉRISATION DE NHR2 ET/OU RUNX1/ETO

(30) Priority: 30.04.2013 EP 13165993
(43) Date of publication of application: 09.03.2016
(73) Proprietor: Heinrich-Heine-Universität Düsseldorf, 40225 Düsseldorf (DE)
(72) Inventor: METZ, Alexander, 35041 Marburg (DE); GOHLKE, Holger, 40591 Düsseldorf (DE); SCHANDA, Julia, 10409 Berlin (DE); WICHMANN, Christian, 82152 Planegg (DE); GREZ, Manuel, 69121 Heidelberg (DE)
(74) Representative: Kutzenberger Wolff & Partner
(86) International application number: PCT/EP2014/058449
(87) International publication number: WO 2014/177464

(56) References cited:
- WO-A1-2009/025781
- WO-A1-2009/071161
- WO-A2-2008/019139
- KAOUD, TAMER S. ET AL: "From in Silico Discovery to Intracellular Activity: Targeting JNK-Protein Interactions with Small Molecules", ACS MEDICINAL CHEMISTRY LETTERS, CODEN: AMCLCT; ISSN: 1948-5875, vol. 3, no. 9, 2012, pages 721-725, XP002728464, DOI: 10.1021/ML300129B
- SALEM BASHANFER ET AL: "siRNAs targeting the ERK2 signaling pathway and AML1/MTG8 fusion gene attenuate the differentiation, proliferation and growth arrest in t(8;21) leukemia", BMC PROCEEDINGS, BIOMED CENTRAL LTD, LONDON UK, vol. 6, no. Suppl 6, 1 October 2012 (2012-10-01), page P29, XP021108066, ISSN: 1753-6561, DOI: 10.1186/1753-6561-6-S6-P29
- DRAKE, NATHAN L. ET AL: "Podophyllotoxin and picropodophyllin. II. The synthesis of an open-chain analog", JOURNAL OF THE AMERICAN CHEMICAL SOCIETY12, CODEN: JACSAT; ISSN: 0002-7863, vol. 77, 1955, pages 1204-1209, XP002728465, DOI: 10.1021/JA01610A034
- METZ, ALEXANDER ET AL: "From Determinants of RUNX1/ETO Tetramerization to Small-Molecule Protein-Protein Interaction Inhibitors Targeting Acute Myeloid Leukemia", JOURNAL OF CHEMICAL INFORMATION AND MODELING, CODEN: JCISD8; ISSN: 1549-9596, vol. 53, no. 9, 19 August 2013 (2013-08-19), pages 2197-2202, XP002728466, DOI: 10.1021/CI400332E
- DATABASE CA [Online] CHEMICAL ABSTRACTS SERVICE, COLUMBUS, OHIO, US; 2009, YANG, DACHENG ET AL: "Preparation of compounds with Mannich base structure and medical application for preventing and/or treating leukemia", retrieved from STN Database accession no. 2009:310904 -& CN 101 381 320 A (SOUTHWEST UNIVERSITY, PEOP. REP. CHINA) 11 March 2009 (2009-03-11)
- DATABASE CA [Online] CHEMICAL ABSTRACTS SERVICE, COLUMBUS, OHIO, US; 2003, MOMEKOV, G. ET AL: "In vitro cytotoxic activity of 4-hydroxycoumarin derivatives in the human", retrieved from STN Database accession no. 2003:468420 & MOMEKOV, G. ET AL: "In vitro cytotoxic activity of 4-hydroxycoumarin derivatives in the human", FARMATSIYA (SOFIA, BULGARIA), CODEN: FMTYA2; ISSN: 0428-0296, vol. 49, no. 1-4, 2002, pages 23-27,
- MOELLER M ET AL: "Antibacterial, antiviral, anti proliferative and apoptosis-inducing properties of Brackenridgea zanguebarica (Ochnaceae)", JOURNAL OF PHARMACY AND PHARMACOLOGY, JOHN WILEY & SONS LTD, LONDON; GB, vol. 58, no. 8, 1 August 2006 (2006-08-01) , pages 1131-1138, XP008087731, ISSN: 0022-3573, DOI: 10.1211/JPP.58.8.0015

## Description

The invention relates to inhibitors of the NHR2 tetramerization from dimers and their use as tumor therapeutics (e.g. against acute myeloid leukemia (AML)), cytostatics, and diagnostic agents.

Chromosomal translocations are frequent events during malignant cell transformation, particularly during leukemogenesis. The translocation t(8;21) is one of the most frequent chromosomal anomalies in acute myeloid leukemia. It involves the RUNX1 gene on chromosome 21 and the ETO gene on chromosome 8. RUNX1 is a transcription factor and belongs to the key regulators of hematopoietic cell differentiation.

The fusion protein RUNX1/ETO has a modular structure. RUNX1/ETO as such is a monomer but forms RUNX1/ETO dimers and RUNX1/ETO tetramers. RUNX1/ETO contains four functional domains, which are generally referred to as nervy homology regions (NHR1 to NHR4).

Several *in vitro* and *in vivo* studies have shown that the nervy homology region 2 (NHR2) is essential for RUNX1/ETO oncogenic activity. The NHR2 domain mediates tetramer formation through hydrophobic and ionic/polar interactions. Two RUNX1/ETO dimers are subsequently positioned on top of each other in a sandwich-like fashion. Similar to RUNX1/ETO monomers, RUNX1/ETO dimers fail to bind efficiently to DNA and to alter expression of RUNX1-dependent genes. RUNX1/ETO dimers do not block myeloid differentiation, are unable to enhance the self-renewal capacity of hematopoietic progenitors, and fail to induce leukemia in a murine transplantation model. The crystal structure of the homo-tetramerization domain of ETO, i.e. the nervy homology region 2 (NHR2), is known from the prior art. In this context, reference can be made, for example, to Y. Liu et al., Cancer Cell, 2006, 9, 249-260; and C. Wichmann et al., Blood, 2010, 116(4), 602-613.

It was an object of the invention to provide compounds that are capable of inhibiting NHR2 tetramerization and/or RUNX1/ETO-tetramerization or the tetramerization of other NHR2-containing proteins that include, but are not limited to, wild type ETO proteins, ETO homologs or the CBFA2T3-GLIS2 fusion proteins, preferably without affecting dimer formation. It was another object of the invention to provide compounds that are useful in the treatment of diseases and disorders that are associated with NHR2 and/or RUNX1/ETO-tetramerization. Further, it was an object of the invention to provide compounds useful as searcher, biotechnological tool or in diagnostics related to NHR2 and/or RUNX1/ETO-tetramerization.

This object has been achieved by the subject-matter of the patent claims.

It has been found that the compounds according to the invention may act as inhibitors of the NHR2 and/or RUNX1/ETO-tetramerization, which is associated with different diseases, such as leukemia, particularly acute myeloid leukemia.

Further, it has been found that the compounds according to the invention inhibit tetramer formation without affecting dimer formation.

A first aspect of the disclosure relates to a compound according to general formula (A), 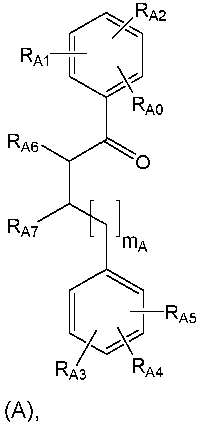 wherein
R_{A0}, R_{A1} and R_{A2}, respectively independently, mean -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
or R_{A0} means -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂; and R_{A1} and R_{A2} are vicinal and together with the carbon atoms to which they are attached form a ring and mean -O-CH₂-O- or -O-CH(R_{X})-O- or -O-C(R_{X})₂-O-;
R_{A3}, R_{A4}, and R_{A5}, respectively independently, mean -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
or R_{A3} means -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHRₓ, -C(=O)N(R_{X})₂, -OH, -ORₓ, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(Rx)₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂; and R_{A4} and R_{A5} are vicinal and together with the carbon atoms to which they are attached form a ring and mean -O-CH₂-O- or -O-CH(R_{X})-O-;
R_{A6} means -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
R_{A7} means -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SRx, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
m_{A} means 0, 1, 2, 3, 4, 5 or 6;
or a physiologically acceptable salt and/or a prodrug thereof.

In a preferred embodiment of the compound according to general formula (A),
R_{A0}, R_{A1} and R_{A2}, respectively independently, mean -H or -OC₁₋₈-aliphatic;
or R_{A0} means -H or -OC₁₋₈-aliphatic; and R_{A1} and R_{A2} are vicinal and together with the carbon atoms to which they are attached form a ring and mean -O-CH₂-O- or -O-CH(C₁₋₈-aliphatic)-O-;
R_{A3}, R_{A4}, and R_{A5}, respectively independently, mean -H or -OC₁₋₈-aliphatic;
or R_{A3} means -H or -OC₁₋₈-aliphatic; and Ra₄ and R_{A5} are vicinal and together with the carbon atoms to which they are attached form a ring and mean -O-CH₂-O- or -O-CH(C₁₋₈-aliphatic)-O-;
R_{A6} means -H or -C₁₋₈-aliphatic;
R_{A7} means -C(=O)OH, -C(=O)OR_{X}, -C(=O)NH₂, -C(=O)NHR_{X}, or -C(=O)N(R_{X})₂; and
m_{A} means 0, 1, 2, 3, or 4.

In another preferred embodiment of the compound according to general formula (A),
R_{A0} means -H;
R_{A1} and R_{A2}, respectively independently, mean -OC₁₋₈-alkyl;
or R_{A1} and R_{A2} are vicinal and together with the carbon atoms to which they are attached form a ring and mean -O-CH₂-O- or -O-CH(C₁₋₈-alkyl)-O-;
R_{A3}, R_{A4}, and R_{A5}, respectively independently, mean -H or -OC₁₋₈-alkyl;
or R_{A3} means -H or -OC₁₋₈-alkyl; and R_{A4} and R_{A5} are vicinal and together with the carbon atoms to which they are attached form a ring and mean -O-CH₂-O- or -O-CH(C₁₋₈-alkyl)-O-;
R_{A6} means -H or -C₁₋₈-alkyl;
R_{A7} means -C(=O)OH or -C(=O)OR_{X}; and
m_{A} means 0, 1, or 2.

In a further preferred embodiment according to general formula (A),
R_{A0} means -H;
R_{A1} and R_{A2}, respectively independently, mean -OCH₃;
or R_{A1} and R_{A2} are vicinal and together with the carbon atoms to which they are attached form a ring and mean -O-CH₂-O- or -O-CH(CH₃)-O-;
R_{A3}, R_{A4}, and R_{A5}, respectively independently, mean -H or -OCH₃;
or R_{A3} means -H; and R_{A4} and R_{A5} are vicinal and together with the carbon atoms to which they are attached form a ring and mean -O-CH₂-O- or -O-CH(CH₃)-O-;
R_{A6} means -H or -CH₃;
R_{A7} means -CO₂H; and
m_{A} means 0 or 1.

Particularly preferred embodiments of the compound according to general formula (A) have the general formulas (A-1) or (A-2):

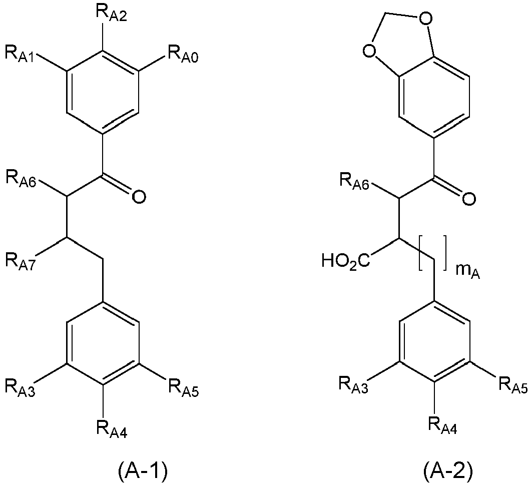

Compounds of the general formula (A-1) that are particularly preferred are those, wherein
R_{A0} means -H;
R_{A1} and R_{A2} together with the carbon atoms to which they are attached form a ring and mean -O-CH(CH₃)-O-;
R_{A3}, R_{A4}, and R_{A5}, respectively independently, mean -H or -OCH₃;
or R_{A3} means -H; and R_{A4} and R_{A5} and together with the carbon atoms to which they are attached form a ring and mean -O-CH₂-O- or -O-CH(CH₃)-O-;
R_{A6} means -H or -CH₃;
R_{A7} means -C(=O)OH or -C(=O)OR_{X}; and
m_{A} means 0 or 1.

Compounds of the general formula (A-1) that are more particularly preferred are those, wherein
R_{A0} means -H;
R_{A1} and R_{A2} together with the carbon atoms to which they are attached form a ring and mean -O-CH(CH₃)-O-;
R_{A3} means -H; and R_{A4} and R_{A5} together with the carbon atoms to which they are attached form a ring and mean -O-CH₂-O-.
R_{A6} means -H;
R_{A7} means -CO₂H;
m_{A} means 0.

Compounds of the general formula (A-2) that are particularly preferred are those, wherein
R_{A3}, R_{A4}, and R_{A5}, respectively independently, mean -H or -OCH₃;
or R_{A3} means -H; and R_{A4} and R_{A5} together with the carbon atoms to which they are attached form a ring and mean -O-CH₂-O- or -O-CH(CH₃)-O-;
R_{A6} means -H or -CH₃;
m_{A} means 0 or 1.

Compounds of the general formula (A-2) that are more particularly preferred are those, wherein
R_{A3}, R_{A4}, and R_{A5}, respectively independently, mean -OCH₃;
or R_{A3} means -H; and R_{A4} and R_{A5} together with the carbon atoms to which they are attached form a ring and mean -O-CH₂-O-;
R_{A6} means -H or -CH₃;
m_{A} means 0 or 1.

Compounds from the following group are most particularly preferred:
2,4-di(benzo[d][1,3]dioxol-5-yl)-4-oxobutanoic acid;
2-(benzo[d][1,3]dioxol-5-yl)-4-(2-methylbenzo[d][1,3]dioxol-5-yl)-4-oxobutanoic acid;
4-(benzo[d][1,3]dioxol-5-yl)-3-methyl-4-oxo-2-(3,4,5-trimethoxybenzyl)butanoic acid;
and the physiologically acceptable salts and/or prodrugs thereof.

Particularly preferred representative compounds according to general formulas (A), (A-1) and (A-2), respectively, are:

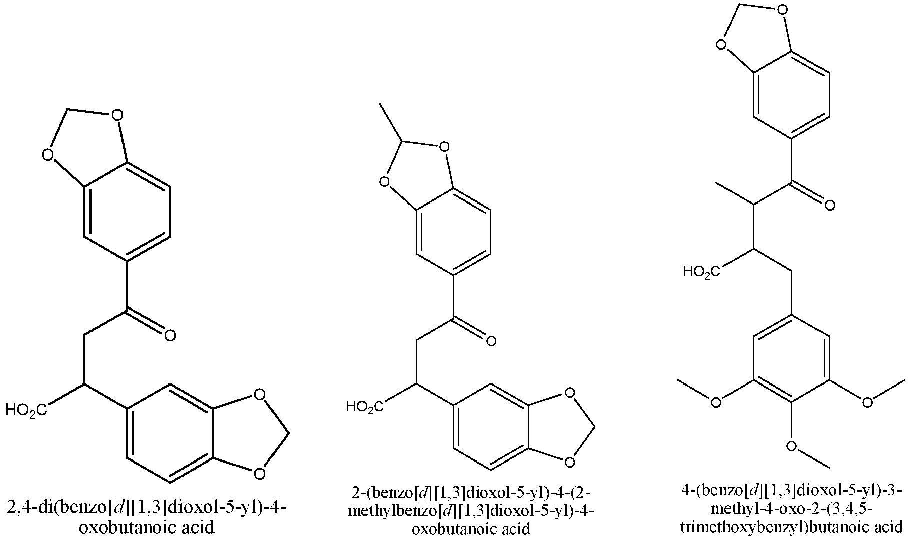

Compounds according to general formulae (B), (C), (D), (E) and (F) are not claimed. Thus, in the following, the definitions of compounds according to general formulae (B), (C), (D), (E) and (F) and references thereto serve information purposes.

A second aspect of the disclosure relates to a compound according to general formula (B) 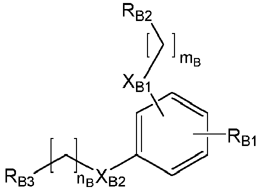 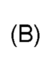 wherein
R_{B1} means -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)Rₓ, -OC(=O)-OR_{X}, -OC(=O)NHR_{X}, -OC(=O)N(R_{X})₂, -SH, -SRx, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)Rₓ, -NHC(=O)OR_{X}, -NHC(=O)NH₂, -NHC(=O)NHR_{X}, -NHC(=O)-N(R_{X})₂, -Si(R_{X})₃ or-PO(OR_{X})₂;
R_{B2} and R_{B3}, respectively independently, mean -F, -CI, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SRx, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHRₓ, -NHC(=O)-N(R_{X})₂, -Si(R_{X})₃ or -PO(OR_{X})₂;
X_{B1} and X_{B2}, respectively independently, mean O, S or NH;
m_{B} means 1, 2, 3, 4, 5, or 6; and
n_{B} means 1, 2, 3, 4, 5, or 6;
or a physiologically acceptable salt and/or a prodrug thereof.

In a preferred embodiment of the compound according to general formula (B),
R_{B1} means -H or -C₁₋₈-aliphatic;
R_{B2} and R_{B3}, respectively independently, mean -C(=O)R_{X}, -C(=O)H, -C(=O)OH, -C(=O)OR_{X}, -C(=O)NH₂, -C(=O)NHR_{X}, or -C(=O)N(R_{X})₂;
X_{B1} and X_{B2}, respectively independently, mean O or S;
m_{B} means 1, 2, 3, or 4; and
n_{B} means 1, 2, 3, or 4.

In another preferred embodiment according to general formula (B),
R_{B1} means -H or -C₁₋₈-alkyl;
R_{B2} and R_{B3}, respectively independently, mean -C(=O)OH or -C(=O)OR_{X};
X_{B1} and X_{B2}, respectively independently, mean O or S;
m_{B} means 1 or 2; and
n_{B} means 1 or 2.

In a further preferred embodiment according to general formula (B),
R_{B1} means -H or -CH₃;
R_{B2} and R_{B3}, respectively independently, mean -C(=O)OH;
X_{B1} and X_{S2}, respectively independently, mean O;
m_{B} means 1; and
n_{B} means 1.

Particularly preferred embodiments of the compound according to general formula (B) have the general formulas (B-1) or (B-2):

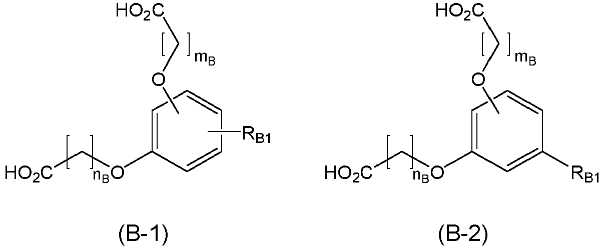

Compounds of the general formulas (B-1) or (B-2) that are particularly preferred are those, wherein
R_{B1} means -H or -CH₃;
m_{B} means 1; and
n_{B} means 1.

Compounds from the following group are most particularly preferred:
2-[2-(carboxymethyloxy)-4-methyl-phenoxy]acetic acid;
2,2'-(1,3-phenylenebis(oxy))diacetic acid;
and the physiologically acceptable salts and/or prodrugs thereof.

Particularly preferred representative compounds according to general formulas (B), (B-1) and (B-2), respectively, are:

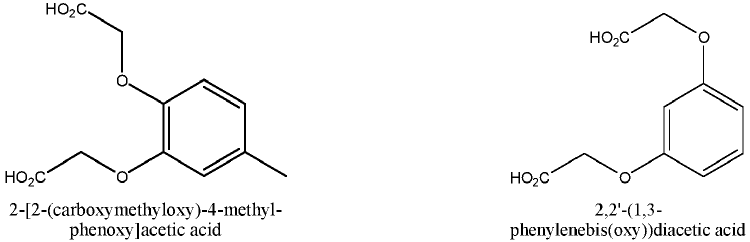

A third aspect of the disclosure relates to a compound according to general formula (C) 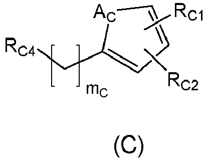 wherein
A_{C} means O, S, or NR_{C3};
R_{C1} means -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or-PO(OR_{X})₂;
R_{C2} means -H or -(CH₂)₁₋₆-X_{C}-R_{X};
R_{C3} means -R_{X};
R_{C4} means -F, -CI, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)Rₓ, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
X_{C} means O, S, or NH; and
m_{C} means 0, 1, 2, 3, 4, 5 or 6;
or a physiologically acceptable salt and/or a prodrug thereof.

In a preferred embodiment of the compound according to general formula (C),
Ac means O or NR_{C3};
R_{C1} means -R_{X};
R_{C2} means -H or -CH₂-X_{C}-CH₂-heteroaryl;
R_{C3} means -(CH₂)₁₋₈-C(=O)OH;
R_{C4} means -C(=O)Rₓ, -C(=O)H, -C(=O)OH, -C(=O)ORₓ, -C(=O)NH₂, -C(=O)NHRₓ, or -C(=O)N(R_{X})₂;
X_{C} means O or S; and
m_{C} means 0, 1, 2, 3, or 4.

In another preferred embodiment according to general formula (C),
Ac means O or NR_{C3};
R_{C1} means -C₁₋₈-aliphatic or -aryl;
R_{C2} means -H, or -CH₂-O-CH₂-heteroaryl or -CH₂-S-CH₂-heteroaryl;
R_{C3} means -(CH₂)₁₋₆-C(=O)OH;
R_{C4} means -C(=O)OH or -C(=O)ORₓ;
X_{C} means O or S; and
m_{C} means 0, 1, or 2.

In a further preferred embodiment according to general formula (C),
Ac means O or NR_{C3};
R_{C1} means -C₁₋₈-alkyl or -phenyl, optionally substituted;
R_{C2} means -H or -CH₂-S-CH₂-heteroaryl;
R_{C3} means -(CH₂)₂-C(=O)OH;
R_{C4} means -C(=O)OH;
X_{C} means S; and
m_{C} means 0 or 2.

Particularly preferred embodiments of the compound according to general formula (C) have the general formula (C-1):

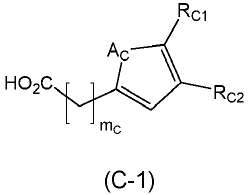

Compounds of the general formula (C-1) that are particularly preferred are those, wherein
A_{C} means O or NR_{C3};
R_{C1} means CH₃ or -phenyl, optionally substituted;
R_{C2} means -H or -CH₂-S-CH₂-heteroaryl, optionally substituted;
R_{C3} means -(CH₂)₂-C(=O)OH; and
m_{C} means 0 or 2.

Compounds of the general formula (C-1) that are more particularly preferred are those, wherein
Ac means O or NR_{C3};
R_{C1} means CH₃ or 4-methoxyphenyl;
R_{C2} means -H or -CH₂-S-CH₂-(2-carboxy-5-methylfuran-4-yl);
R_{C3} means -(CH₂)₂-C(=O)OH; and
m_{C} means 0 or 2.

Compounds from the following group are most particularly preferred:
3,3'-(5-(4-methoxyphenyl)-1 H-pyrrole-1,2-diyl)dipropanoic acid;
5,5'-dimethyl-4,4'-(sulfanediylbis(methyl))-di(furan-2-carboxylic acid);
and the physiologically acceptable salts and/or prodrugs thereof.

Particularly preferred representative compounds according to general formulas (C) and (C-1), respectively, are:

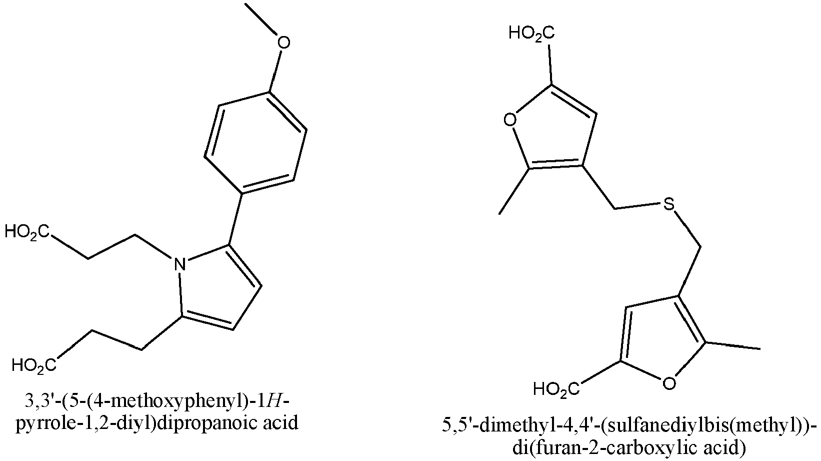

A fourth aspect of the disclosure relates to a compound according to general formula (D) 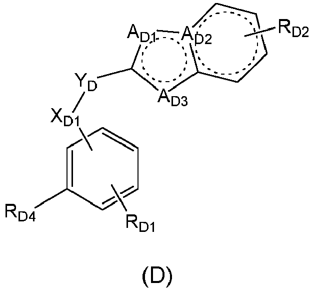 wherein
X_{D1} means O, S, or NR_{D3};
X_{D2} means O or S;
Y_{D} means -(CH₂)₀₋₆- or -C(=O)-(CH₂)₁₋₆-X_{D2}-;
A_{D1} means N, NH or CH;
A_{D2} means N or C;
A_{D3} means N or CH;
R_{D1} means -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or-PO(OR_{X})₂;
R_{D2} means -H, -F, -CI, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
R_{D3} means -H, -F, -CI, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
R_{D4} means -C(=O)R_{X}, -C(=O)H, -C(=O)OH, -C(=O)OR_{X}, -C(=O)NH₂, -C(=O)NHR_{X}, or -C(=O)N(R_{X})₂;
or a physiologically acceptable salt and/or a prodrug thereof.

In a preferred embodiment of the compound according to general formula (D),
X_{D1} means O or NR_{D3};
X_{D2} means O or S;
Y_{D} means -(CH₂)₁₋₄- or -C(=O)-(CH₂)₁₋₄-X_{D2}-;
A_{D1} means N, NH or CH;
A_{D2} means N or C;
A_{D3} means N or CH;
with the proviso that 2 of A_{D1}, A_{D2} and A_{D3} mean N and NH, respectively, while the other means C and CH, respectively;
R_{D1} means -H, -F, -CI, -Br, -I, -CN, or -NO₂;
R_{D2} means -H or -R_{X};
R_{D3} means -H or -R_{X}; and
R_{D4} means -C(=O)OH or -C(=O)OR_{X};

In another preferred embodiment according to general formula (D),
X_{D1} means O or NR_{D3};
X_{D2} means S;
Y_{D} means -CH₂- or -C(=O)-(CH₂)-S-;
A_{D1} means N or CH;
A_{D2} means N or C;
A_{D3} means N or CH;
with the proviso that 2 of A_{D1}, A_{D2} and A_{D3} mean N and NH, respectively, while the other means C and CH, respectively;
R_{D1} means -H or -CI;
R_{D2} means -H;
R_{D3} means -H; and
R_{D4} means -CO₂H.

Particularly preferred embodiments of the compound according to general formula (D) have the general formula (D-1):

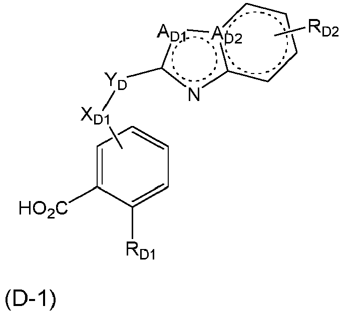

Compounds of the general formula (D-1) that are particularly preferred are those, wherein
X_{D1} means O or NR_{D3};
Y_{D} means -CH₂-, -S-(CH₂)-C(=O)- or -C(=O)-(CH₂)-S-;
A_{D1} means NH or CH;
A_{D2} means N or C;
with the proviso that A_{D1} means CH or NH, while A_{D2} means N or C, respectively;
R_{D1} means -H or -CI;
R_{D2} means -H;
R_{D3} means -H.

Compounds from the following group are most particularly preferred:
2-(imidazo[1,2-a]pyridin-2-ylmethoxy)benzoic acid;
5-(2-(1 H-benzo[d]imidazol-2-ylthio)acetamido)-2-chlorobenzoic acid;
and the physiologically acceptable salts and/or prodrugs thereof.

Particularly preferred representative compounds according to general formulas (D) and (D-1), respectively, are:

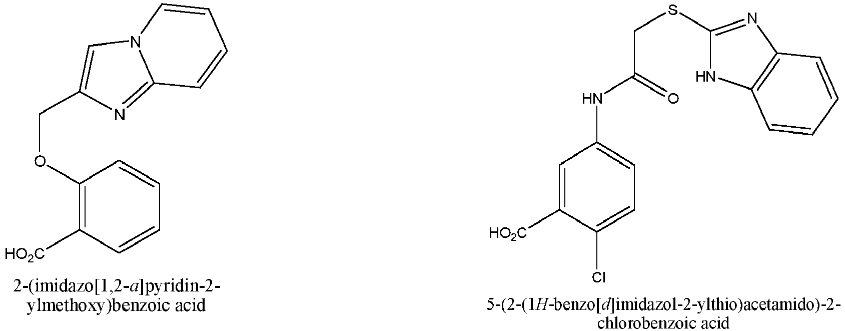

A fifth aspect of the disclosure relates to a compound according to general formula (E) 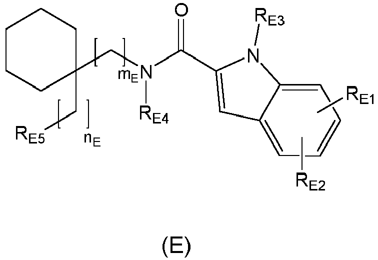 wherein
R_{E1} and R_{E2}, respectively independently, mean -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or-PO(OR_{X})₂;
R_{E3} and R_{E4}, respectively independently, mean -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
R_{E5} means -F, -CI, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, NHC(=O)NH₂, -NHC(=O)NHR_{X}, -NHC(=O)-N(R_{X})₂, -Si(R_{X})₃ or -PO(OR_{X})₂;
m_{E} means 0, 1, 2, 3, 4, 5, or 6; and
n_{E} means 0, 1, 2, 3, 4, 5, or 6;
or a physiologically acceptable salt and/or a prodrug thereof.

In a preferred embodiment of the compound according to general formula (E),
R_{E1} and R_{E2}, respectively independently, mean -OC₁₋₈-aliphatic;
R_{E3} and R_{E4}, respectively independently, mean -H or -R_{X};
R_{E5} means -C(=O)R_{X}, -C(=O)H, -C(=O)OH, -C(=O)OR_{X}, -C(=O)NH₂, -C(=O)NHR_{X}, or C(=O)N(R_{X})₂;
m_{E} means 0, 1, 2, 3, or 4; and
n_{E} means 0, 1, 2, 3, or 4;

In another preferred embodiment according to general formula (E),
R_{E1} and R_{E2}, respectively independently, mean -OC₁₋₈-alkyl;
R_{E3} and R_{E4}, respectively independently, mean -H or -R_{X};
R_{E5} means -C(=O)OH or -C(=O)OR_{X};
m_{E} means 0, 1, or 2; and
n_{E} means 0, 1, or 2.

In a further preferred embodiment according to general formula (E),
R_{E1} and R_{E2}, respectively independently, mean -OCH₃;
R_{E3} and R_{E4}, respectively independently, mean -H;
R_{E5} means -C(=O)OH;
m_{E} means 1; and
n_{E} means 1.

Particularly preferred embodiments of the compound according to general formula (E) have the general formula (E-1):

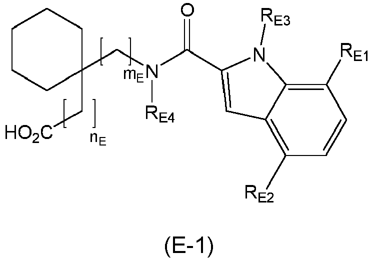

Compounds of the general formula (E-1) that are particularly preferred are those, wherein
R_{E1} and R_{E2}, respectively independently, mean -OCH₃;
R_{E3} and R_{E4}, respectively independently, mean -H;
m_{E} means 1; and
n_{E} means 1.

Compounds from the following group are most particularly preferred:
2-(1-((4,7-dimethoxy-1 H-indole-2-carboxamido)methyl)cyclohexyl)acetic acid;
and the physiologically acceptable salts and/or prodrugs thereof.

A particularly preferred representative compound according to general formulas (E) and (E-1), respectively, is:

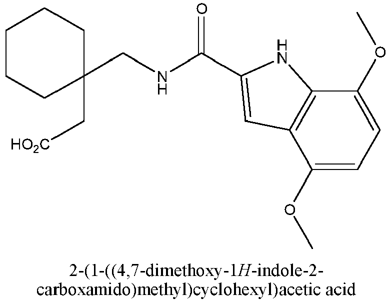

A sixth aspect of the disclosure relates to a compound according to general formula (F) 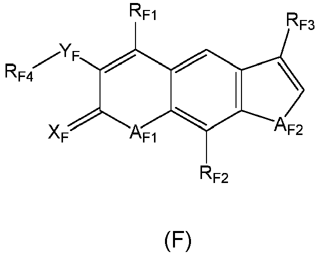 wherein
R_{F1} means -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or-PO(OR_{X})₂;
R_{F2} means -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
R_{F3} means -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
R_{F4} means -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
R_{F5}, R_{F6} and R_{F7}, respectively independently, mean -H, -F, -Cl, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
X_{F} means O, S, or NR_{FS};
Y_{F} means -(CH₂)₁₋₆C(=O)NH-(CH₂)₁₋₆-;
A_{F1} means O, S, or NR_{F6};
A_{F2} means O, S, or NR_{F7};
or a physiologically acceptable salt and/or a prodrug thereof.

In a preferred embodiment of the compound according to general formula (F),
R_{F1} means -C₁₋₈-aliphatic;
R_{F2} means -C₁₋₈-aliphatic;
R_{F3} means -C₁₋₈-aliphatic;
R_{F4} means -C(=O)R_{X}, -C(=O)H, -C(=O)OH, -C(=O)OR_{X}, -C(=O)NH₂, -C(=O)NHR_{X}, or -C(=O)N(R_{X})₂;
R_{F5}, R_{F6} and R_{F7}, respectively independently, mean -H;
X_{F} means O;
Y_{F} means -(CH₂)₁₋₆C(=O)NH-(CH₂)₁₋₆-;
A_{F1} means O; and
A_{F2} means O.

In another preferred embodiment according to general formula (F),
R_{F1} means -C₁₋₈-alkyl;
R_{F2} means -C₁₋₈-alkyl;
R_{F3} means -C₁₋₈-alkyl;
R_{F4} means -C(=O)OH or -C(=O)OR_{X};
R_{F5}, R_{F6} and R_{F7}, respectively independently, mean -H;
X_{F} means O;
Y_{F} means -(CH₂)₁₋₄C(=O)NH-(CH₂)₁₋₄-;
A_{F1} means O; and
A_{F2} means O.

In a further preferred embodiment according to general formula (F),
R_{F1} means -CH₃;
R_{F2} means -CH₃;
R_{F3} means -C(CH₃)₃;
R_{F4} means -CO₂H;
R_{F5}, R_{F6} and R_{F7}, respectively independently, mean -H;
X_{F} means O;
Y_{F} means -CH₂C(=O)NH-(CH₂)₂-;
A_{F1} means O; and
A_{F2} means O.

Particularly preferred embodiments of the compound according to general formula (F) have the general formula (F-1):

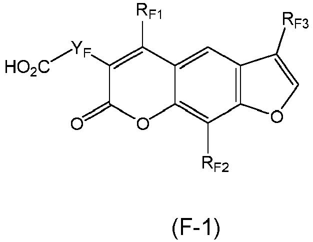

Compounds of the general formula (F-1) that are particularly preferred are those, wherein
R_{F1} means -CH₃;
R_{F2} means -CH₃;
R_{F3} means -C(CH₃)₃; and
Y_{F} means -CH₂C(=O)NH-(CH₂)₂-;

Compounds from the following group are most particularly preferred:
3-(2-(3-tert-butyl-5,9-dimethyl-7-oxo-7H-furo[3,2-g]chromen-6-yl)acetamido)propanoic acid;
and the physiologically acceptable salts and/or prodrugs thereof.

A particularly preferred representative compound according to general formulas (F) and (F-1), respectively, is:

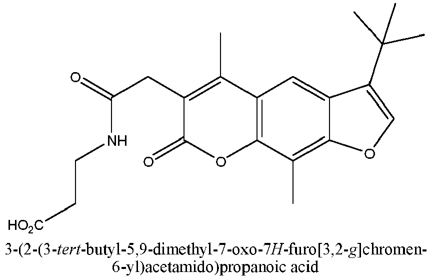

For the purpose of the specification, RUNX1/ETO is the fusion protein containing the DNA-binding domain (Runt, RHD) of the RUNX1 transcription factor but lacking the C-terminal transactivation sequence being replaced by almost the entire ETO protein. A compound inhibiting RUNX1/ETO-tetramerization is a compound that reduces the tendency of RUNX1/ETO to form tetramers. For the purpose of the specification, NHR2 is a functional domain of RUNX1/ETO, namely the nervy homology region 2. A compound inhibiting NHR2 is a compound that
a) reduces the tendency of NHR2 to form tetramers, and/or
b) reduces the tendency of NHR2-containing proteins that include, but are not limited to, wild type ETO proteins, homologs of ETO proteins and CBFA2T3-GLIS2 fusion proteins (Gruber et al., Cancer Cell, 2012, 22, 683-697; C. Thiollier et al., The Journal of Experimental Medicine 2012, 209(11), 2017-2031) to form tetramers, and/or
c) interferes with the onset and/or maintenance of AML and other NHR2 dependent leukemias, and/or
d) interferes with the role of NHR2-containing proteins in other disorders or diseases that include, but are not limited to, cancer or a hematopoietic disease, preferably leukemia, particularly preferably selected from the group consisting of myeloid leukemia, acute myeloid leukemia, promyeloid leukemia, acute promyeloid leukemia, promyelocytic leukemia, acute promyelocytic leukemia, megakaryoblastic leukemia and acute megakaryoblastic leukemia, and/or
e) enables a personalized therapy of NHR2-dependend disorders or diseases, and/or
f) interferes with blocking of myeloid differentiation caused by NHR2 tetramerization, and/or
g) interferes with the proliferation of RUNX1/ETO-dependent cells that include, but are not limited to, SKNO-1 cells, and/or
h) interferes with the enhanced self-renewal capacity of hematopoietic progenitors, and/or
i) interferes with the induction or maintenance of disorders or diseases that include, but are not limited to, cancer or a hematopoietic disease, preferably leukemia, particularly preferably selected from the group consisting of myeloid leukemia, acute myeloid leukemia, promyeloid leukemia, acute promyeloid leukemia, promyelocytic leukemia, acute promyelocytic leukemia, megakaryoblastic leukemia and acute megakaryoblastic leukemia in mammals (Y. Liu et al., Cancer Cell, 2006, 9, 249-260; C. Wichmann et al., Blood, 2010, 116(4), 602-613; and C. Wichmann et al., Cancer Research, 2007, 67, 2280-2289).

For further details, reference is made to e.g. D.G. Tenen, Nat Rev Cancer, 2003; 3(2): 89-101; J. Zhang et al., Science, 2004; 305(5688): 1286-1289; Metz et al., Current Pharmaceutical Design, 2012, 18, 4630-4647.

For the purpose of the specification, in the definitions of the compounds according to general formulas (A), (B), (C), (D), (E) and (F),
in each case Rx, respectively independently, means -C₁₋₈-aliphatic, -C₃₋₁₂-cycloaliphatic, -aryl, heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁₋₈-aliphatic, -C₃₋₈-cycloaliphatic-aryl or -C₃₋₈-cycloaliphatic-heteroaryl;
in each case "aliphatic", respectively independently, means a branched or unbranched, saturated or a mono- or polyunsaturated, unsubstituted or mono- or polysubstituted, aliphatic hydrocarbon residue;
in each case "cycloaliphatic", respectively independently, means a saturated or a mono- or polyunsaturated, unsubstituted or mono- or polysubstituted, alicyclic, mono- or multicyclic hydrocarbon residue;
in each case with respect to "aliphatic" and "cycloaliphatic", "mono- or polysubstituted", respectively independently, means the mono- or polysubstitution of one or more hydrogen atoms by -F, -CI, -Br, -I, -CN, -NO₂, -CHO, =O, -Rx, -C(=O)R_{X}, -C(=O)H, -C(=O)OH, -C(=O)OR_{X}, -C(=O)NH₂, -C(=O)NHR_{X}, -C(=O)N(R_{X})₂, -OH, -OR_{X}, -OC(=O)H, -OC(=O)R_{X}, -OC(=O)-OR_{X}, -OC(=O)NHR_{X}, -OC(=O)N(R_{X})₂, -SH, -SRx, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
in each case "aryl", respectively independently, means a carbocyclic ring system with at least one aromatic ring, but without heteroatoms in this ring, wherein, if necessary, the aryl residues can be condensed with further saturated, (partially) unsaturated or aromatic ring systems, and each aryl residue can be present in unsubstituted or mono- or polysubstituted form, wherein the aryl substituents can be the same or different and in any desired and possible position of the aryl;
in each case "heteroaryl", respectively independently, means a 5-, 6- or 7-membered cyclic aromatic residue, which contains 1, 2, 3, 4 or 5 heteroatoms, wherein the heteroatoms, the same or different, are nitrogen, oxygen or sulphur, and the heterocycle can be unsubstituted or mono- or polysubstituted; wherein in the case of the substitution on the heterocycle the substituents can be the same or different and can be in any desired and possible position of the heteroaryl; and wherein the heterocycle can also be part of a bi- or polycyclic system;
in each case with respect to "aryl" and "heteroaryl", "mono- or polysubstituted", respectively independently, means the mono- or polysubstitution of one or more hydrogen atoms of the ring system by substituents selected from the group comprising -F, -CI, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)-N(R_{X})₂, -OH, -O(CH₂)₁₋₂O-, -OR_{X}, -OC(=O)H, -OC(=O)R_{X}, -OC(=O)OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NH-C(=O)NH₂, -NHC(=O)NHR_{X}, -NHC(=O)-N(R_{X})₂, -Si(R_{X})₃ and -PO(OR_{X})₂; wherein if necessary N-ring atoms present can be respectively oxidized;
in the form of a single stereoisomer or mixture thereof, the free compounds and/or their physiologically compatible salts and/or prodrugs and/or solvates.

In the combination of different residues, e.g. R_{A3}, R_{A4}, and R_{A5}, and also the combination of residues at substituents thereof such as e.g. -ORₓ, -SRₓ, -S(=O)₁₋₂-R_{X} or -C(=O)OR_{X}, a substituent, e.g. Rₓ, can assume different meanings within a substance for two or more residues, e.g. R_{A3}, R_{A4}, and R_{A5}.

For the purposes of the description hydrocarbon residues are divided into aliphatic hydrocarbon residues and aromatic hydrocarbon residues.

Aliphatic hydrocarbon residues are themselves divided into non-cyclic aliphatic hydrocarbon residues (= "aliphatic") and cyclic aliphatic hydrocarbon residues, i.e. alicyclic hydrocarbon residues (= "cycloaliphatic"). Cycloaliphatic compounds can be monocyclic or multicyclic. Alicyclic hydrocarbon residues ("cycloaliphatic") comprise both pure aliphatic carbocycles and aliphatic heterocycles, i.e. - unless expressly specified - "cycloaliphatic" comprises pure aliphatic carbocycles (e.g. cyclohexyl), pure aliphatic heterocycles (e.g. piperidyl or piperazyl) and also non-aromatic, multicyclic, possibly mixed, systems (e.g. decalinyl, decahydroquinolinyl).

Aromatic hydrocarbons are themselves divided into carbocyclic aromatic hydrocarbons (="aryl") and heterocyclic aromatic hydrocarbons (="heteroaryl").

The classification of multicyclic, at least partially aromatic systems preferably depends on whether at least one aromatic ring of the multicyclic system has at least one heteroatom (usually N, O or S) in the ring. If at least one such heteroatom is present in this ring, this is preferably a "heteroaryl" (even if a further carbocyclic aromatic or non-aromatic ring with or without heteroatom is possibly present as additionally present cycle of the multicyclic system); if such a heteroatom is not present in any of the possibly several aromatic rings of the multicyclic system, then this is preferably "aryl" (even if a ring heteroatom is present in a possibly additionally present non-aromatic cycle of the multicyclic system).

Therefore, the following priority in the classification applies within the cyclic substituents: heteroaryl > aryl > cycloaliphatic.

For the purposes of the description monovalent and multivalent, i.e. bivalent, hydrocarbon residues are not distinguished between conceptually, i.e. depending on the context, "C₁₋₈-aliphatic" covers e.g. -C₁₋₈-alkyl, -C₁₋₈-alkenyl and -C₁₋₈-alkinyl, as well as e.g. -C₁₋₈-alkylene-, -C₁₋₈-alkenylene- and C₁₋₈-alkinylene.

Aliphatic means preferably respectively a branched or unbranched, saturated or a mono- or polyunsaturated, unsubstituted or mono- or polysubstituted, aliphatic hydrocarbon residue. Where aliphatic is mono- or polysubstituted, the substituents are selected independently of one another from the group comprising -F, -CI, -Br, -I, -CN, -NO₂, -CHO, =O, -Rx, -C(=O)R_{X}, -C(=O)H, -C(=O)OH, -C(=O)OR_{X}, -C(=O)NH₂, -C(=O)NHR_{X}, -C(=O)N(R_{X})₂, -OH, -ORx, -OC(=O)H, -OC(=O)R_{X}, -OC(=O)-OR_{X}, -OC(=O)NHR_{X}, -OC(=O)N(R_{X})₂, -SH, -SRx, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂.

Thus, "aliphatic" covers acyclic saturated or unsaturated hydrocarbon residues that can be branched or straight-chain, i.e. alkanyls, alkenyls and alkinyls. In this case, alkenyls have at least one C=C double bond and alkinyls have at least one C=C triple bond. Preferred unsubstituted monovalent aliphatics comprise -CH₃, -CH₂CH₃, -CH₂CH₂CH₃, -CH(CH₃)₂, -CH₂CH₂CH₂CH₃, -CH(CH₃)CH₂CH₃, -CH₂CH(CH₃)₂, -C(CH₃)₃, -CH₂CH₂CH₂-CH₂CH₃ and -CH₂CH₂CH₂CH₂CH₂CH₃; but also -CH=CH₂, -C≡CH, -CH₂CH=CH₂, -CH=CHCH₃, -CH₂C=CH, -C≡CCH₃ and -CH=CHCH=CH₂. Preferred unsubstituted bivalent aliphatics comprise -CH₂-, -CH₂CH₂-, -CH₂CH(CH₃)-, -CH(CH₃)-CH₂-, -CH₂CH₂CH₂-, -CH(CH₃)CH₂CH₂-, -CH₂CH(CH₃)-CH₂-, -CH₂CH₂CH(CH₃)-, -CH-(CH₂CH₃)CH₂- and -CH₂CH₂-CH₂CH₂-; but also -CH=CH-, -C≡C-, -CH₂CH=CH-, -CH=CHCH₂-, -CH₂C≡C- and -C≡CCH₂-. Preferred substituted monovalent aliphatics comprise -CH₂F, -CHF₂, -CF₃, -CH₂CF₃, -CF₂CF₃, -CH₂OH, -CH₂CH₂OH, -CH₂CHOHCH₃, -CH₂OCH₃, -CH₂CH₂OCH₃ and -CH₂N(CH₃)₂. Preferred substituted bivalent aliphatics comprise -CF₂-, -CF₂CF₂-, -CH₂CHOH-, -CHOHCH₂- and -CH₂CHOHCH₂-. -Methyl-, -ethyl-, -n-propyl- and -n-butyl- are particularly preferred.

Cycloaliphatic means preferably respectively a saturated or a mono- or polyunsaturated, unsubstituted or mono- or polysubstituted, aliphatic (i.e. not aromatic), mono- or multicyclic hydrocarbon residue. The number of ring-carbon atoms preferably lies in the specified range (i.e. a "C₃₋₁₂-cycloaliphatic" preferably has 3, 4, 5, 6, 7, 8, 9, 10, 11 or 12 ring-carbon atoms). For the purposes of the description "C₃₋₁₂-cycloaliphatic" is preferably a cyclic hydrocarbon with 3, 4, 5, 6, 7, 8, 9, 10, 11 or 12 ring-carbon atoms, saturated or unsaturated, but not aromatic, wherein possibly one or two carbon atoms are replaced independently of one another by a heteroatom S, N or O. Where cycloalkyl is mono- or polysubstituted, the substituents are selected independently of one another from the group comprising -F, -CI, -Br, -I, -CN, -NO₂, -CHO, =O, -Rx, -C(=O)R_{X}, -C(=O)H, -C(=O)OH, -C(=O)OR_{X}, -C(=O)NH₂, -C(=O)NHR_{X}, -C(=O)N(R_{X})₂, -OH, -ORx, -OC(=O)H, -OC(=O)R_{X}, -OC(=O)-OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂.

Preferably, C₃₋₁₂-cycloaliphatic is selected from the group comprising cyclopropyl, cyclobutyl, cyclopentyl, cyclohexyl, cycloheptyl, cyclooctyl, cyclononyl, cyclodecyl, cycloundecyl, cyclododecyl, cyclopentenyl, cyclohexenyl, cycloheptenyl, cyclooctenyl, cyclononenyl, cyclodecenyl, cycloundecenyl, cyclododecenyl, but also tetrahydropyranyl, dioxanyl, dioxolanyl, morpholinyl, piperidinyl, piperazinyl, pyrazolinonyl and pyrrolidinyl.

In association with "aliphatic" or "cycloaliphatic", "mono- or polysubstituted" is preferably understood to mean the mono- or polysubstitution, e.g. the mono-, di-, tri- or 4-substitution, of one or more hydrogen atoms by -F, -CI, -Br, -I, -OH, -OC₁₋₈-alkyl, -OC(=O)C₁₋₈-alkyl, -SH, -NH₂, -NHC₁₋₈-alkyl, -N(C₁₋₈-alkyl)₂, -C(=O)OC₁₋₈-alkyl or -C(=O)OH. Particularly preferred substituents are -F, -CI, -OH, -SH, -NH₂ and -C(=O)OH.

Polysubstituted residues are understood to be those residues that are polysubstituted, e.g. twice or three times either at different or at the same atoms, e.g. three times at the same C-atom, as in the case of -CF₃ or -CH₂CF₃, or at different sites, as in the case of -CH(OH)-CH=CH-CHCl₂. The polysubstitution can occur with the same or with different substituents. A substituent may also be substituted itself. Thus, -Oaliphatic also covers -OCH₂CH₂O-CH₂CH₂OH, amongst others. It is preferred if aliphatic or cycloaliphatic is substituted with -F, -CI, -Br, -I, -CN, -CH₃, -C₂H₅, -NH₂, -NO₂, -SH, -CF₃, -OH, -OCH₃, -OC₂H₅ or -N(CH₃)₂. It is most particularly preferred if aliphatic or cycloaliphatic is substituted with -OH, -OCH₃ or -OC₂H₅.

Aryl preferably respectively independently stands for a carbocyclic ring system with at least one aromatic ring, but without heteroatoms in this ring, wherein the aryl residues can possibly be condensed with further saturated, (partially) unsaturated or aromatic ring systems and each aryl residue can be present in unsubstituted or mono- or polysubstituted form, wherein the aryl substituents are the same or different and can be in any desired and possible position of the aryl. Preferred aryls are phenyl, naphthyl, anthracenyl, phenanthrenyl, fluoroanthenyl, fluoroenyl, indanyl and tetralinyl. Phenyl and naphthyl are particularly preferred. Where aryl is mono- or polysubstituted, the aryl substituents can be the same or different and be in any desired and possible position of the aryl, and are selected independently of one another from the group comprising -F, -CI, -Br, -I, -CN, -NO₂, -CHO, =O, -R_{X}, -C(=O)R_{X}, -C(=O)H, -C(=O)OH, -C(=O)OR_{X}, -C(=O)NH₂, -C(=O)NHR_{X}, -C(=O)-N(R_{X})₂, -OH, -O(CH₂)₁₋₂O-, -OR_{X}, -OC(=O)H, -OC(=O)R_{X}, -OC(=O)OR_{X}, -OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NH-C(=O)NH₂, -NHC(=O)NHR_{X}, -NHC(=O)-N(R_{X})₂, -Si(R_{X})₃ and -PO(OR_{X})₂; wherein if necessary N-ring atoms present can be respectively oxidized. Preferred substituted aryls are 2-fluorophenyl, 3-fluorophenyl, 4-fluorophenyl, 2,3-difluorophenyl, 2,4-difluorophenyl, 3,4-difluorophenyl, 2-chlorophenyl, 3-chlorophenyl, 4-chlorophenyl, 2,3-dichlorophenyl, 2,4-dichlorophenyl, 3,4-dichlorophenyl, 2-methoxy-phenyl, 3-methoxy-phenyl, 4-methoxy-phenyl, 2,3-dimethoxy-phenyl, 2,4-dimethoxy-phenyl, 3,4-dimethoxy-phenyl, 2-methyl-phenyl, 3-methyl-phenyl, 4-methyl-phenyl, 2,3-dimethyl-phenyl, 2,4-dimethyl-phenyl and 3,4-dimethyl-phenyl.

Heteroaryl preferably stands for a 5-, 6- or 7-membered cyclic aromatic residue that contains 1, 2, 3, 4 or 5 heteroatoms, wherein the heteroatoms, the same or different, are nitrogen, oxygen or sulphur, and the heterocycle can be unsubstituted or mono- or polysubstituted; wherein in the case of the substitution on the heterocycle, the substituents can be the same or different and can be in any desired and possible position of the heteroaryl; and wherein the heterocycle can also be part of a bi- or polycyclic system. "Heteroaryl" is preferably selected from the group comprising pyrrolyl, indolyl, furyl (furanyl), benzofuranyl, thienyl (thiophenyl), benzothienyl, benzothiadiazolyl, benzooxadiazolyl, benzothiazolyl, benzooxazolyl, benzotriazolyl, benzodioxolanyl, benzodioxanyl, phthalazinyl, pyrazolyl, imidazolyl, thiazolyl, oxazolyl, isoxazoyl, pyridinyl, pyridazinyl, pyrimidinyl, pyrazinyl, pyranyl, indazolyl, purinyl, indolizinyl, quinolinyl, isoquinolinyl, quinazolinyl, carbazolyl, phenazinyl, phenothiazinyl or oxadiazolyl, wherein the bonding can occur via any desirable and possible ring member of the heteroaryl residue. Where heteroaryl is mono- or polysubstituted, the heteroaryl substituents can be the same or different and can be in any desirable and possible position of the heteroaryl, and are selected independently of one another from the group comprising -F, -CI, -Br, -I, -CN, -NO₂, -CHO, =O, -Rx, -C(=O)R_{X}, -C(=O)H, -C(=O)OH, -C(=O)OR_{X}, -C(=O)NH₂, -C(=O)NHR_{X}, -C(=O)-N(R_{X})₂, -OH, -O(CH₂)₁₋₂O-, -ORx, -OC(=O)H, -OC(=O)R_{X}, -OC(=O)OR_{X}, -OC(=O)NHR_{X}, -OC(=O)N(R_{X})₂, -SH, -SRx, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NH-C(=O)NH₂, -NHC(=O)NHR_{X}, -NHC(=O)-N(R_{X})₂, -Si(R_{X})₃ and -PO(OR_{X})₂; wherein if necessary N-ring atoms present can be respectively oxidized.

Regarding "aryl" or "heteroaryl", "mono- or polysubstituted" are understood to mean the mono- or polysubstitution, e.g. di-, tri-, 4- or 5- substitution, of one or more hydrogen atoms of the ring system.

Particularly preferred are the (hetero)aryl substituents selected independently of one another from -F, -CI, -Br, -I, -CN, -CHO, -CO₂H, -NH₂, -NO₂, -NHR_{X}, -N(Rₓ)₂, -N⁺(Rₓ)₃, -N⁺(Rₓ)₂O⁻, -SH, -SRₓ, -OH, -ORₓ, -C(=O)Rₓ, -CO₂Rₓ, -C(=O)NH₂, -C(=O)NHRₓ, -C(=O)N(Rₓ)₂, -S(=O)₁₋₂Rₓ, -S(=O)₂NH₂, -SO₃H, =O or -Rₓ. Preferred substituents are -F, -CI, -Br, -I, -OH, -OC₁₋₈-alkyl, -O-C(=O)-C₁₋₈-alkyl, -SH, -NH₂, -NHC₁₋₈-alkyl, -N(C₁₋₈-alkyl)₂, -C(=O)OC₁₋₈-alkyl or -C(=O)OH. Particularly preferred substituents are -F, -CI, -OH, -SH, -NH₂ and -C(=O)OH.

Unless expressly stated otherwise, residues having more than a single binding partner can be attached in any direction. For example, the residue "-S-(CH₂)-C(=O)-" which is attached to binding partners B₁ and B₂ can be present in either direction, B₁-S-(CH₂)-C(=O)-B₂ or B₁-C(=O)-(CH₂)-S-B₂.

Particularly preferred are compounds according to general formulas (A), (B), (C), (D), (E) and (F), respectively, wherein
- R_{A0} means -H; R_{A1} and R_{A2} together with the carbon atoms to which they are attached form a ring and mean -O-CH₂-O- or -O-CH(CH₃)-O-; R_{A6} means -H or -CH₃; R_{A7} means -CO₂H; R_{A3}, R_{A4}, and R_{A5}, respectively independently, mean -OCH₃; or R_{A3} means -H; and R_{A4} and R_{A5} together with the carbon atoms to which they are attached form a ring and mean -O-CH₂-O-; and m_{A} means 0 or 1;
- R_{B1} means -H or -CH₃; R_{B2} and R_{B3} mean CO₂H; X_{B1} and X_{B2} mean -O-; m_{B} means 1; and n_{B} means 1;
- A_{C} means O or NR_{C3}; R_{C1} means CH₃ or 4-methoxyphenyl; R_{C2} means -H or -CH₂-S-CH₂-(5-methylfuran-2-carboxylic acid); R_{C3} means -(CH₂)₂-C(=O)OH; R_{C4} means CO₂H; m_{C} means 0 or 2;
- X_{D1} means O or NR_{D3}; X_{D2} means S; Y_{D} means -CH₂-, -X_{D2}-(CH₂)-C(=O)-; or -C(=O)-(CH₂)-X_{D2}-; A_{D1} means NH or CH; A_{D2} means N or C; A_{D3} means N; with the proviso that A_{D1} means CH or NH, while A_{D2} means N or C, respectively; R_{D1} means -H or -CI; R_{D2} means -H; R_{D3} means -H; R_{D4} means CO₂H;
- R_{E1} and R_{E2}, respectively independently, mean -OCH₃; R_{E3} and R_{E4}, respectively independently, mean -H; R_{E5} means -C(=O)OH; m_{E} means 1; and n_{E} means 1;
- R_{F1} and R_{F2} mean -CH₃; R_{F3} means -C(CH₃)₃; R_{F4} means -CO₂H ; Y_{F} means -CH₂C(=O)NH-(CH₂)₂-; X_{F} means =O; A_{F1} and A_{F2} mean -O-.

The compounds according to the invention can be present in the form of a single stereoisomer or mixture thereof, the free compounds and/or their physiologically compatible salts and/or solvates.

The compounds according to the invention can be chiral or achiral, depending on the substitution pattern.

If the compounds according to the invention are chiral, then they are preferably present as racemate or a mixture of stereoisomers or diastereomers or in enriched form of an enantiomer. In a preferred embodiment the enantiomer excess (ee) of the S-enantiomer amounts to at least 50% ee, more preferred at least 75% ee, more preferred at least 90% ee, most preferred at least 95% ee, and in particular at least 99% ee. In another preferred embodiment, the enantiomer excess (ee) of the R-enantiomer amounts to at least 50% ee, more preferred at least 75% ee, more preferred at least 90% ee, most preferred at least 95% ee, and in particular at least 99% ee.

Suitable methods for separating the enantiomers are known to the person skilled in the art. Preparative HPLC on chiral stationary phases and conversion into diastereomeric intermediates can be given as examples. The conversion into diastereomeric intermediates can occur, for example, as salt formation by means of chiral, enantiomer-pure acids. After separation of the diastereomers thus formed, the salt can then be converted into the free base or another salt again.

Unless expressly specified, each reference to the compounds according to the invention covers all isomers in pure form and admixture with one another (e.g. stereoisomers, diastereomers, enantiomers) in any desired mixture ratio.

Unless expressly specified, each reference to the compounds according to the invention covers the free compounds (i.e. the forms that are not present in the form of salt) and all physiologically compatible salts.

For the purposes of the description, physiologically compatible salts of the compounds according to the invention are present as salts with anions or acids of the respective compound with inorganic or organic acids, which are physiologically compatible - in particular on application in humans and/or mammals.

Examples of physiologically compatible salts of specific acids are salts of: hydrochloric acid, hydrobromic acid, sulphuric acid, methane sulphonic acid, formic acid, acetic acid, oxalic acid, succinic acid, malic acid, tartaric acid, mandelic acid, fumaric acid, lactic acid, citric acid, glutamic acid, saccharinic acid, monomethyl sebacic acid, 5-oxo-proline, hexane-1-sulphonic acid, nicotinic acid, 2-, 3- or 4-aminobenzoic acid, 2,4,6-trimethyl benzoic acid, α-liponic acid, acetylglycine, acetylsalicylic acid, hippuric acid and/or aspartic acid. The hydrochloride, citrate and hemicitrate are particularly preferred.

Physiologically compatible salts with cations or bases are salts of the respective compound - as anion with at least one, preferably inorganic, cation, which are physiologically compatible - in particular on application in humans and/or mammals. Particularly preferred are the salts of the alkali and earth alkali metals, also ammonium salts, but in particular (mono-) or (di-) sodium, (mono-) or (di-) potassium, magnesium or calcium salts.

Prodrugs of the compounds according to the invention are also disclosed and are such which are converted *in vivo* to the pharmacologically active compound. The most common prodrugs are carboxylic acid esters, e.g. acetates, ethyl esters. For further details and a detailed list of prodrug concepts that is not to be construed as limiting the scope of possible prodrugs, reference is made to e.g. J. Rautio, Nat Rev Drug Discov, 2008, 7, 255-270.

The compounds according to the invention are defined by substituents, e.g. by R_{A3}, R_{A4}, and R_{A5} (substituents of the first generation), which are themselves possibly substituted (substituents of the second generation). Depending on the definition, these substituents of the substituents can themselves be substituted again (substituents of the third generation). If, for example, R_{A3} = -Rₓ, wherein -Rₓ = -C₁₋₈-aliphatic (substituent of the first generation), then -C₁₋₈-aliphatic can itself be substituted, e.g. with -ORₓ, wherein Rₓ = -aryl (substituent of the second generation). This gives the functional group -C₁₋₈-aliphatic-O-aryl. -Aryl can then in turn be substituted again, e.g. with -Cl (substituent of the third generation). This then gives overall the functional group -C₁₋₈-aliphatic-O-aryl-Cl.

The compounds according to the invention act, for example, as inhibitors of the NHR2 and/or RUNX1/ETO-tetramerization which is associated with different diseases, and therefore they are suitable as pharmaceutical active substance in a medication.

Another aspect of the invention relates to the compounds according to the invention as described above as medicaments.

Another aspect of the invention relates to pharmaceutical compositions or pharmaceutical dosage forms comprising the compounds according to the invention as described above.

Preferably, the pharmaceutical compositions comprise a compound according to the invention as described above, and a pharmaceutically acceptable carrier. The term "pharmaceutically acceptable carrier," as used herein, means a non-toxic, inert solid, semisolid or liquid filler, diluent, encapsulating material or formulation auxiliary of any type.

Some examples of materials which may serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; cocoa butter and suppository waxes; oils such as peanut oil, cottonseed oil, safflower oil, sesame oil, olive oil, corn oil and soybean oil; glycols; such a propylene glycol; esters such as ethyl oleate and ethyl laurate; agar; buffering agents; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as releasing agents, coloring agents, coating agents, sweetening, flavoring and perfuming agents, preservatives and antioxidants may also be present in the composition, according to the judgment of one skilled in the art of formulations.

The pharmaceutical compositions may be administered to subjects (e.g., humans and other mammals) orally, rectally, parenterally, intravaginally, intracisternally, intraperitoneally, topically (as by powders, ointments or drops), bucally, extracorporeally, e.g. by dialysis, or as an oral or nasal spray. The term "parenterally," as used herein, refers to modes of administration, including intravenous, intramuscular, intraperitoneal, intrasternal, subcutaneous, intraarticular injection and infusion.

Pharmaceutical compositions for parenteral injection comprise pharmaceutically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (polyethylene glycol, propylene glycol, glycerol, and the like, and suitable mixtures thereof), vegetable oils (such as olive oil) and injectable organic esters such as ethyl oleate, or suitable mixtures thereof. Suitable fluidity of the composition may be maintained, for example, by the use of a coating such as lecithin, by the maintenance of the required particle size in the case of dispersions, and by the use of surfactants.

These compositions may also contain adjuvants, such as preservative agents, wetting agents, emulsifying agents, and dispersing agents. Prevention of the action of microorganisms may be ensured by various antibacterial and antifungal agents, for example, parabens, phenol, chlorobutanol, sorbic acid, and the like. It also may be desirable to include isotonic agents, for example, sugars, sodium chloride and the like. Prolonged absorption of the injectable pharmaceutical form may be brought about by the use of agents delaying absorption, for example, aluminum monostearate and gelatin.

In some cases, in order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug may depend upon its rate of dissolution, which, in turn, may depend upon crystal size and crystalline form. Alternatively, a parenterally administered drug form may be administered by dissolving or suspending the drug in an oil vehicle.

Suspensions, in addition to the active compounds, may contain suspending agents, for example, polyoxyethylene sorbitol, ethoxylated isostearyl alcohols, and sorbitan esters, microcrystalline cellulose, aluminum metahydroxide, bentonite, agar-agar, tragacanth, and mixtures thereof.

If desired, and for more effective distribution, the compounds may be incorporated into slow-release or targeted-delivery systems such as polymer matrices, liposomes, and microspheres. They may be sterilized, for example, by filtration through a bacteria-retaining filter or by incorporation of sterilizing agents in the form of sterile solid compositions, which may be dissolved in sterile water or some other sterile injectable medium immediately before use.

Injectable depot forms are made by forming microencapsulated matrices of the drug in biodegradable polymers such as polylactide-polyglycolide. Depending upon the ratio of drug to polymer and the nature of the particular polymer employed, the rate of drug release may be controlled. Depot injectable formulations also are prepared by entrapping the drug in liposomes or microemulsions which are compatible with body tissues.

The injectable formulations may be sterilized, for example, by filtration through a bacterial-retaining filter or by incorporating sterilizing agents in the form of sterile solid compositions which may be dissolved or dispersed in sterile water or other sterile injectable medium just prior to use.

Injectable preparations, for example, sterile injectable aqueous or oleaginous suspensions may be formulated according to the known art using suitable dispersing or wetting agents, suspending agents and the like. The sterile injectable preparation also may be a sterile injectable solution, suspension or emulsion in a nontoxic, parenterally acceptable diluent or solvent such as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose, any bland fixed oil may be employed including synthetic mono- or diglycerides. In addition, fatty acids such as oleic acid are used in the preparation of injectables.

Solid dosage forms for oral administration include, but are not limited to, capsules, tablets, pills, powders, and granules. In such solid dosage forms, one or more compounds is mixed with at least one inert pharmaceutically acceptable carrier such as sodium citrate or dicalcium phosphate and/or a) fillers or extenders such as starches, lactose, sucrose, glucose, mannitol, and salicylic acid; b) binders such as carboxymethylcellulose, alginates, gelatin, polyvinylpyrrolidinone, sucrose, and acacia; c) humectants such as glycerol; d) disintegrating agents such as agar-agar, calcium carbonate, potato or tapioca starch, alginic acid, certain silicates, and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay; and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using lactose or milk sugar as well as high molecular weight polyethylene glycols.

The solid dosage forms of tablets, dragees, capsules, pills, and granules may be prepared with coatings and shells such as enteric coatings and other coatings well-known in the pharmaceutical formulating art. They optionally may contain opacifying agents and also may be of a composition that they release the active ingredient(s) only, or preferentially, in a certain part of the intestinal tract in a delayed manner. Examples of materials useful for delaying release of the active agent may include polymeric substances and waxes.

Compositions for rectal or vaginal administration are preferably suppositories which may be prepared by mixing the compounds with suitable non-irritating carriers such as cocoa butter, polyethylene glycol or a suppository wax which are solid at ambient temperature but liquid at body temperature and therefore melt in the rectum or vaginal cavity and release the active compound.

Liquid dosage forms for oral administration may include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions may also include adjuvants such as wetting agents, emulsifying and suspending agents, sweetening, flavoring, and perfuming agents.

Suspensions, in addition to the active compounds, may contain suspending agents, for example, ethoxylated isostearyl alcohols, polyoxyethylene sorbitol and sorbitan esters, microcrystalline cellulose, aluminum metahydroxide, bentonite, agar-agar, tragacanth, and mixtures thereof.

If desired, and for more effective distribution, the compounds may be incorporated into slow-release or targeted-delivery systems such as polymer matrices, liposomes, and microspheres. They may be sterilized, for example, by filtration through a bacteria-retaining filter or by incorporation of sterilizing agents in the form of sterile solid compositions, which may be dissolved in sterile water or some other sterile injectable medium immediately before use.

Dosage forms for topical or transdermal administration of a compound include ointments, pastes, creams, lotions, gels, powders, solutions, sprays, inhalants or patches. A desired compound is admixed under sterile conditions with a pharmaceutically acceptable carrier and any needed preservatives or buffers as may be required. Ophthalmic formulation, eardrops, eye ointments, powders and solutions are also contemplated as being within the scope of this disclosure.

The ointments, pastes, creams and gels may contain, in addition to an active compound, animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays may contain, in addition to the compounds, lactose, talc, silicic acid, aluminum hydroxide, calcium silicates and polyamide powder, or mixtures of these substances. Sprays additionally may contain customary propellants such as chlorofluorohydrocarbons.

Compounds also may be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes may be used. The present compositions in liposome form may contain, in addition to the compounds, stabilizers, preservatives, and the like. The preferred lipids are the natural and synthetic phospholipids and phosphatidylcholines (lecithins) used separately or together. Methods to form liposomes are known in the art.

Dosage forms for topical administration of a compound according to the invention as described above include powders, sprays, ointments and inhalants. The active compound is mixed under sterile conditions with a pharmaceutically acceptable carrier and any needed preservatives, buffers or propellants. Ophthalmic formulations, eye ointments, powders and solutions are also possible. Aqueous liquid compositions may also be useful.

Another aspect of the invention relates to the compounds according to the invention as described above for use in the treatment or the prevention of a disorder or disease that is associated with NHR2 and/or RUNX1/ETO-tetramerization.

Another aspect of the invention relates to the use of inhibitors of NHR2 and/or RUNX1/ETO-tetramerization, which are preferably non-peptidic and/or which preferably have a molecular weight of at most 2000 g/mol, more preferably at most 1000 g/mol, still more preferably at most 750 g/mol and in particular at most 500 g/mol, for the manufacture of a medicament for the treatment or the prevention of a disorder or disease that is associated with NHR2 and/or RUNX1/ETO-tetramerization, preferably leukemia, more preferably myeloid leukemia, in particular acute myeloid leukemia.

Another aspect of the invention relates to the use of inhibitors of NHR2 and/or RUNX1/ETO-tetramerization, which are preferably non-peptidic and/or which preferably have a molecular weight of at most 2000 g/mol, more preferably at most 1000 g/mol, still more preferably at most 750 g/mol and in particular at most 500 g/mol, for the treatment or the prevention of a disorder or disease that is associated with NHR2 and/or RUNX1/ETO-tetramerization, preferably leukemia, more preferably myeloid leukemia, in particular acute myeloid leukemia.

Another aspect of the invention relates to the use of the compounds according to the invention as described above for the manufacture of a medicament for the treatment or the prevention of a disorder or disease that is associated with NHR2 and/or RUNX1/ETO-tetramerization.

Another aspect of the invention relates to a method of treating or preventing a disorder or disease that is associated with NHR2 and/or RUNX1/ETO-tetramerization, the method comprising the administration of a therapeutically effective amount of a compound or a physiologically acceptable salt or a pharmaceutical composition or a pharmaceutical dosage form according to the invention as described above to a subject in need thereof.

In a preferred embodiment the disorder or disease that is associated with NHR2 and/or RUNX1/ETO-tetramerization is cancer or a hematopoietic disease, preferably leukemia.

Preferably, the leukemia is myeloid leukemia, particularly preferably the leukemia is selected from the group consisting of acute myeloid leukemia, promyeloid leukemia, acute promyeloid leukemia, promyelocytic leukemia, acute promyelocytic leukemia, megakaryoblastic leukemia and acute megakaryoblastic leukemia.

In a preferred embodiment, the compounds according to the invention are for administration once daily. In another preferred embodiment, the compounds or physiologically acceptable salts or pharmaceutical compositions or pharmaceutical dosage forms according to the invention are for administration multiple daily, in particular twice daily or thrice daily, or more often or continuously infused.

In a preferred embodiment, the compounds according to the invention are administered orally. In another preferred embodiment, the compounds according to the invention are administered parenterally. In another preferred embodiment, the compounds according to the invention are administered systemically, locally or extracorporeally.

Another aspect of the invention relates to the use of the compounds according to the invention for measuring the inhibition of NHR2 and/or RUNX1/ETO-tetramerization in an assay preferably selected from the group that includes, but is not limited to, ABCD, EMSA, ELISA and cross-linking assay.

Preferably, the ABCD assay is an avidin-biotin complex DNA-binding assay (ABCD) using 5'-biotinylated oligonucleotides corresponding to the RUNX1 binding sequence within RUNX3 and PU.1 and mutants of these binding sequences. Preferably, the mutant of these binding sequences is R3mut, which is incapable of RUNX1 binding. Preferably, the ABCD assay is as further described in the experimental section.

For the purpose of the specification, RUNX3 is the RUNX3 promoter and PU.1 is the PU.1 promoter. For the purpose of the specification, R3 is a double-stranded RUNX3 oligonucleotide corresponding to the RUNX1 binding sequences derived from the RUNX3 promoter sequence that is optionally biotinylated for detection in assays. The 5'-biotinylated oligonucleotide corresponding to the RUNX1 binding sequences within RUNX3 (R3) and its mutant incapable of RUNX1 binding (R3mut) were: R3: 5'-AGG GCC TGG CCT TGT GGT TCT GTG GTT GAG GGA CCA GGC-3'; R3mut: 5' AGG GCC TGG CCT TGT TAG TCT GTT AGT GAG GGA CCA GGC. For the purpose of the specification, RUNX1/ETO-m5 is a mutant RUNX1/ETO protein in which five amino acids of the NHR2 domain (amino acid 482-548 in RUNX1/ETO) are mutated to alanine (positions 498, 502, 533, 536 and 540; C. Wichmann et al., Blood, 2010, 116(4), 602-613) that way interfering with the formation of the NHR2 and/or RUNX1/ETO tetramerization.

Preferably, for the ABCD assay, the R3 or R3mut oligonucleotide was annealed to the corresponding antisense unmodified oligonucleotide to generate the RUNX1 binding site. The 5'-biotinylated oligonucleotide within the human PU.1 promoter region (PU.1) was: 5'-TGG GCC GCT GTG CGG TGC CTG TGG TAA TGG GCT GT-3'. Biotinylated double-stranded oligonucleotides were mixed with nuclear extracts from vector transfected 293T cells in binding buffer (25 mM HEPES, pH 7.5, 50 mM KCI, 1 mM EDTA, pH 8.0, 10 mM MgCI2, 5% Glycerol, 1 mM DTT) in the presence of 1% NP40 and 100 ng of salmon sperm DNA and incubated. Streptavidin magnetic beads (Dynabeads M-280 Streptavidin, Invitrogen) were then added to the mixture and incubated for one hour. After washing three times, the bound proteins were eluted from beads in SDS-PAGE sample buffer and resolved on SDS-PAGE followed by immunoblotting with anti-HA (Covance), anti-ETO (C-20, Santa Cruz) or anti-Flag (F3165, Sigma, Germany) antibody.

Preferably, for electrophoretic mobility shift assays (EMSA), 293T cells are transfected with vectors for RUNX1/ETO or RUNX1/ETO-m5. Cell extracts were prepared by lysing transfected cells in cell lysis buffer (50 mM Tris pH 7.4, 100 mM NaCl, 0.1% Triton X-100, 1 mM DTT, 1 mM EDTA, 10% glycerol) followed by centrifugation to remove insoluble material. Labeling of the probe and separation by PAGE was performed as described previously (G. Huang et al., Blood, 2004; 103, 3200-3207). EMSA were carried out with equal amounts of protein including a high salt buffer (10 mM Tris pH 7.5, 1 mM EDTA pH 8.0, 1 mM EGTA pH 8.0, 0.4 M NaCl, 20% Glycerol, 1 mM DTT) and salmon sperm DNA as an unspecific competitor. A RUNX1-high-affinity oligo (J. A. Mertz et al., Journal of Virology, 2001, 75, 2174-2184) was used as a probe (5'-CAT GGC CCT TTG CGG TTA GTT AC-3'). Preferably, the EMSA assay is as further described in the experimental section.

Preferably, for enzyme-linked immunosorbent assays (ELISA), wells of a 96 well plate were coated with 100 ng streptavidin (Dianova) in PBS over night at 4°C. A double-stranded biotinylated RUNX3 oligonucleotide (100 ng, R3 = 5'-AGG GCC TGG CCT TGT GGT TCT GTG GTT GAG GGA CCA GGC-3') was bound to streptavidin in PBS containing 0.1% BSA and 0.05% Tween-20 for 2 h at RT on the 96 well plate. RUNX1-containing proteins were incubated with synthetic peptides or chemical compounds in binding buffer (25 mM HEPES, pH 7.5, 50 mM KCI, 1 mM EDTA, pH 8.0, 10 mM MgCI2, 5% glycerol, 1 mM DTT) in the presence of 1 µg salmon sperm and 1% IGEPAL CA-630 for 5 h and subsequently added to the streptavidin-bound R3 oligonucleotide for 15 min on the 96 well plate. Binding of RUNX1-containing proteins to the R3 oligonucleotide was detected with a primary anti-myc antibody (9E10, Santa Cruz) and a secondary HRP-coupled antibody (donkey anti-mouse IgG-HRP, Santa Cruz). ELISA was developed using the Sure Blue TMB Microwell Peroxidase substrate (KLP), and the reaction was stopped with sulfuric acid (1 N). Absorption at 450 nm and 650 nm was measured with the Spectra Max 340, Molecular Devices. Preferably, the ELISA assay is as further described in the experimental section.

Preferably, for the cross-linking assay, purified NHR2 protein or NHR2-containing protein (5 µM) was incubated with the peptides at 4°C for 1 h. The BS³ (Bis[sulfosuccinimidyl] suberate) crosslink-reaction was performed at a final concentration of 1 mM for 30 min at RT. Tris-HCI (0.05 M, pH 7.4) was added to the reaction mix and incubated for 10 min to stop the reaction. Protein-oligomerization was analyzed by western blotting. For protein detection, the membrane was incubated with a primary anti-myc antibody (9E10, Santa Cruz) and a secondary HRP-coupled antibody (goat anti-mouse IgG-HRP, Santa Cruz). Preferably, the cross-linking assay is as further described in the experimental section.

Another aspect of the invention relates to the use of the compounds according to the invention as described above with respect to assays for the diagnosis of diseases associated with the tetramerization of NHR2 and/or NHR2 containing proteins.

Yet, another aspect of the invention relates to the use of the compounds according to the invention as described above with respect to assays for screening for tetramerization of NHR2 and/or of NHR2 containing proteins *in vitro, ex vivo, in vivo,* in mammals and humans, and the like.

Yet, another aspect of the invention relates to the use of the compounds according to the invention as described above as a reference compound or competing binder/inhibitor in the applications as described above, i.e. as a searcher, biotechnological tool or in diagnostics for inhibition of NHR2 and/or RUNX1/ETO-tetramerization.

The following examples further illustrate the invention but are not to be construed as limiting its scope:

### Structure-based identification of inhibitors of the NHR2 tetramerization

An analysis of the previously identified five hot spots suggested D533, E536, and W540 as the most suitable template motif (C. Wichmann et al., Blood, 2010). This template motif was used together with the structural knowledge of the location and orientation of these residues' side chains in the crystal structure in a virtual screening (VS) for inhibitors mimicking the hot spot interactions. Finally, 80 compounds were selected for experimental testing.

The inhibition of NHR2 tetramerization was measured *in vitro* by tetramer-dependent binding of a RUNX1-NHR2 protein construct to an immobilized oligonucleotide derived from the RUNX3 promoter sequence in ELISA and ABCD assays. Selectivity for NHR2 was demonstrated by the reduced inhibition observed when replacing the NHR2 domain of the construct with the homologous BCR tetramerization domain (RUNX1-BCR protein).

Furthermore, the influence of the inhibitors on the viability of RUNX1/ETO-dependent SKNO-1 cells was determined; as a control RUNX1/ETO-independent U937 cells were used.

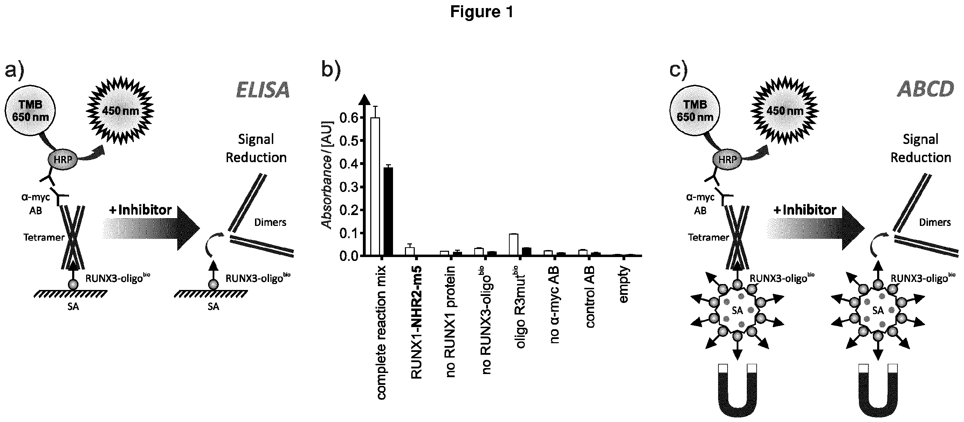

The principle of the biochemical assays for measuring inhibiton of NHR2 tetramerization is illustrated in Figure 1.

NHR2 tetramerization and its inhibition was measured in a purpose-built sandwich ELISA (enzyme-linked immunosorbent assay; Figure 1a).
i) The RUNX1-NHR2 tetramer specifically binds to the biotinylated RUNX3-oligonucleotide (RUNX3-oligo^{bio}) that is immobilized on a streptavidine coated surface (SA). Disrupting the RUNX1-NHR2 tetramer into dimers leads to a release of the RUNX1-NHR2 protein from the surface. Antibody-conjugated horseradish peroxidase (HRP) binds to a primary anti-myc antibody (a-myc AB) that binds to the immobilized myc-labeled RUNX1-NHR2 tetramer. HRP catalyzes the conversion of 3,3',5,5'-tetramethylbenzidine (TMB) to 3,3',5,5'-tetramethylbenzidine diimine. The ELISA signal was quantified photometrically as the difference of absorbance at 650 nm and 450 nm relative to the corrsponding difference of absorbance in the absence of an inhibitor (--).
ii) The specificity of this ELISA assay was validated by comparing signal reduction related to tetramer dependent surface binding of the RUNX1-NHR2 protein (white bars) or the RUNX1-BCR protein (black bars; both Figure 1b) under the following conditions:
   (i) presence of the complete reaction mix but no inhibitor;
   (ii) presence of hot spot alanine mutations in the NHR2 domain (RUNX1-NHR2-m5; N.A. for RUNX1-BCR);
   (iii) absence of a RUNX1-containing protein (no RUNX1 protein);
   (iv) absence of RUNX3-oligo^{bio} (no RUNX3-oligo^{bio});
   (v) presence of a mutated RUNX3-oligo^{bio} (oligo R3mut^{bio});
   (vi) absence of the primary anti-myc antibody (no α-myc AB);
   (vii) replacement of the anti-myc antibody by a control antibody (control AB); or (viii) absence of all components from the reaction mix (empty).
iii) The inhibitory effect of selected compounds was confirmed with a complementary ABCD (avidin, biotin, complex, DNA) assay (Figure 1c) that differs from the ELISA in that the SA coated surface is replaced by SA magnetic beads for segregation of bound tetramer followed by SDS-PAGE, immunoblotting, and signal quantification consistent with the ELISA.

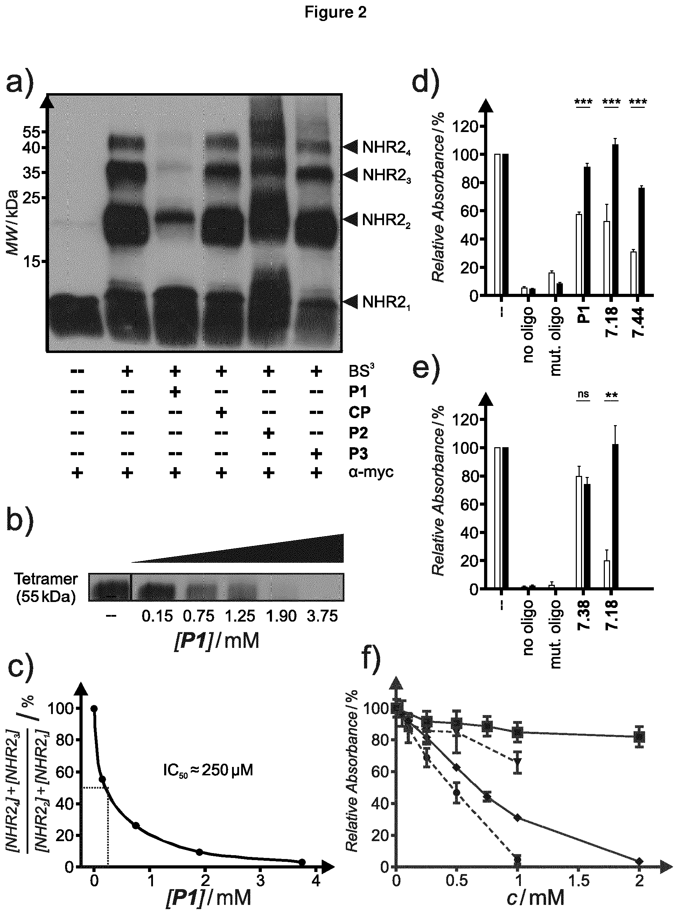

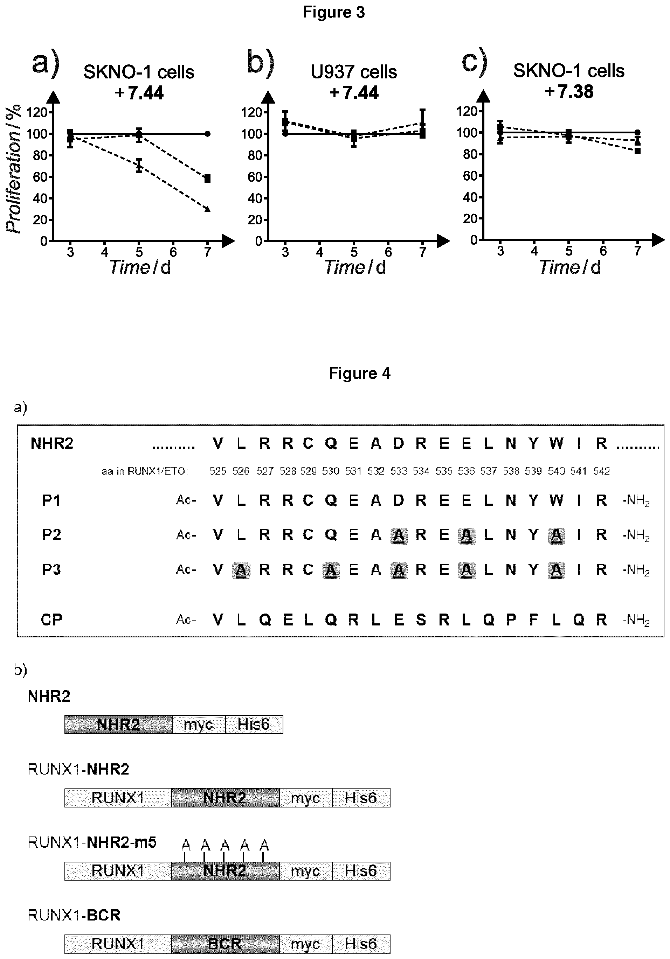

Figure 2 illustrates the inhibition of NHR2 tetramerization. The following conclusions can be drawn:
a) Peptide **P1,** but neither **P2, P3** nor the unrelated peptide **CP** inhibit NHR2 tetramerization as shown by the reduction of BS³ cross-linked NHR2 oligomers (*c*= 1100 µM);
b) The inhibition by **P1** is dose-dependent;
c) with *IC₅₀ ≈* 250 µM;
d) ELISA; and
e) ABCD experiments show that **P1, 7.18,** and **7.44,** but not **7.38,** selectively inhibit tetramer-dependent binding of the RUNX1-NHR2 protein (white bars) to an immobilized RUNX3 oligonucleotide while binding of the RUNX1-BCR protein (black bars) is not inhibited (*[P1]* = 500 µM and *[PPIM]* = 2000 µM in ELISA; *c* = 1100 µM in ABCD assay);
f) dose-dependent inhibition of RUNX1-NHR2 tetramerization in the ELISA by **P1** (●; *IC₅₀* = 460 µM) and **7.44** (◆ ;*IC₅₀* = 680 µM) but neither by **P3** (▼) nor by **7.38** (■);
standard deviations for *n* ≥ 2; ns: not significant; **: 0.01 > p ≥ 0.001; ***: p < 0.0001 (unpaired t-test).
iv) The inhibitory effect of selected compounds in the ELISA assay was demonstrated by comparing signal reduction related to tetramer dependent surface binding of the RUNX1-NHR2 protein (white bars) or the RUNX1-BCR protein (black bars; both Figure 2d) under the following conditions:
   (i) presence of the complete reaction mix but no inhibitor (--);
   (ii) absence of RUNX3-oligo^{bio} (no oligo);
   (iii) presence of a mutated RUNX3-oligo^{bio} (mut. oligo);
   (iv) presence of the complete reaction mix and the 18mer peptide inhibitor **P1** at a final concentration of 500 µM (**P1;** sequence given in Figure 4a)
   (v) presence of the complete reaction mix and the inhibitor **7.18** at a final concentration of 2000 µM;
   (vi) presence of the complete reaction mix and the inhibitor **7.44** at a final concentration of 2000 µM.
v) The inhibitory effect of selected compounds in the ABCD assay was demonstrated by comparing signal reduction related to tetramer dependent surface binding of the RUNX1-NHR2 protein (white bars) or the RUNX1-BCR protein (black bars; both Figure 2e) under the following conditions:
   (i) presence of the complete reaction mix but no inhibitor (--);
   (ii) absence of RUNX3-oligo^{bio} (no oligo);
   (iii) presence of a mutated RUNX3-oligo^{bio} (mut. oligo);
   (iv) presence of the complete reaction mix and the non-inhibitory reference substance **7.38** at a final concentration of 1100 µM;
   (v) presence of the complete reaction mix and the inhibitor **7.18** at a final concentration of 1100 µM;

Figure 4 shows:
a) Sequences of the peptides used in NHR2 inhibition assays aligned to the wild type NHR2 sequence (alanine mutations highlighted); corresponding sequence positions according to RUNX1/ETO numbering are denoted for the NHR2 sequence; all peptides were C-terminally acetylated and N-terminally amidated;
b) protein constructs used in ELISA and ABCD assays; the wild type NHR2 tetramerization domain or the homologous BCR tetramerization domain (highlighted) are optionally enclosed by an N-terminal RUNX1 domain and C-terminal c-myc and His6 tags; the alanine mutations in the RUNX1-NHR2-m5 protein (indicated by the "A"s above the NHR2 domain) correspond to RUNX1/ETO positions 526, 530, 533, 536, and 540, in accord with the alanine mutations of **P2** depicted in a).
   vi) The dose-dependency of the inhibitory effect of selected compounds in the ELISA assay was demonstrated by measuring the signal reduction related to tetramer dependent surface binding of the RUNX1-NHR2 protein (Figure 2f) under the following conditions:
      (i) presence of the complete reaction mix and the 18mer peptide inhibitor **P1** at different concentrations (●; sequence of **P1** given in Figure 4a);
      (ii) presence of the complete reaction mix and the inhibitor **7.44** at different concentrations (◆);
      (iii) presence of the complete reaction mix and the non-inhibitory 18mer peptide **P3** at different concentrations (▼; sequence of **P3** given in Figure 4a);
      (iv) presence of the complete reaction mix and the non-inhibitory reference substance **7.38** at different concentrations (■);
   vii) The inhibitory effect of selected compounds in the ELISA assay was quantified by means of *IC₅₀* values and the inhibition in the presence of an inhibitor at a specific concentration relative to the absence of that particular inhibitor (relative inhibition in %). Each measurement was carried out in the presence of the complete reaction mix and in the presence of an inhibitor or reference compound from the group consisting of **P1, P3, 2b, 5.4, 5.8, 5.9, 5.17, 5.18, 7.3, 7.4, 7.18, 7.38,** and **7.44** (Table 1 below).

The effect of the inhibition of NHR2 tetramerization on cells was measured in a cell survical assay (Figure 3). It becomes clear that NHR2 inhibitor **7.44** specifically reduces proliferation of RUNX1/ETO-dependent cells; SKNO-1 cell or U937 cells were treated with 1 µM (■) or 10 µM (▲) of **7.44** or **7.38** or no PPIM (●). The following conclusions can be drawn:
a) RUNX1/ETO-dependent SKNO-1 cells were severely affected by **7.44;**
b) while RUNX1/ETO-independent U937 cells were unaffected; **7.38,** inactive in vitro, did not affect SKNO-1 cells; proliferation measured by XTT assay; standard deviations for *n* ≥ 3.
NHR2 dependent SKNO-1 cells and NHR2 independent U937 cells were cultured. Cell proliferation and viability were measured with the cell proliferation kit II (XTT, Roche Applied Science). The XTT assay was performed at days 3, 5, and 7. 3000 cells per well were cultured in a 96 well plate and daily treated with compounds under the following conditions:
(i) SKNO-1 cells were daily treated with 0 µM of compound **7.44** (●; Figure 3a);
(ii) SKNO-1 cells were daily treated with 1 µM of compound **7.44** (■; Figure 3a);
(iii) SKNO-1 cells were daily treated with 10 µM of compound **7.44** (▲; Figure 3a);
(iv) U937 cells were daily treated with 0 µM of compound **7.44** (●; Figure 3b);
(v) U937 cells were daily treated with 1 µM of compound **7.44** (■; Figure 3b);
(vi) U937 cells were daily treated with 10 µM of compound **7.44** (▲; Figure 3b);
(vii) SKNO-1 cells were daily treated with 0 µM of compound **7.38** (●; Figure 3c);
(viii) SKNO-1 cells were daily treated with 1 µM of compound **7.38** (■; Figure 3c); or
(ix) SKNO-1 cells were daily treated with 10 µM of compound **7.38** (▲; Figure 3c).

For the purpose of the specification, **P1** is an inhibitory 18mer peptide (sequence given in Figure 4a), **P2, P3,** and **CP** are non-inhibitory 18mer peptides (sequences given in Figure 4a), and **2b****, 5.4, 5.8, 5.9, 5.17, 5.18, 7.3, 7.4, 7.18, 7.38,** and **7.44** are chemical compounds, wherein
- **2b** is 2-(imidazo[1,2-a]pyridin-2-ylmethoxy)benzoic acid;
- **5.4** is 5-(2-(1H-benzo[d]imidazol-2-ylthio)acetamido)-2-chlorobenzoic acid;
- **5.8** is 3,3'-(5-(4-methoxyphenyl)-1*H*-pyrrole-1,2-diyl)dipropanoic acid;
- **5.9** is 5,5'-dimethyl-4,4'-(sulfanediylbis(methyl))-di(furan-2-carboxylic acid);
- **5.17** is 3-(2-(3-tert-butyl-5,9-dimethyl-7-oxo-7H-furo[3,2-g]chromen-6-yl)acetamido)propanoic acid;
- **5.18** is 2-(1-((4,7-dimethoxy-1H-indole-2-carboxamido)methyl)cyclohexyl)acetic acid;
- **7.3** is 2-[2-(carboxymethyloxy)-4-methyl-phenoxy]acetic acid;
- **7.4** is 2,2'-(1,3-phenylenebis(oxy))diacetic acid;
- **7.18** is 4-(benzo[d][1,3]dioxol-5-yl)-3-methyl-4-oxo-2-(3,4,5-trimethoxybenzyl)butanoic acid;
- **7.38** is (E)-2-(2-carboxyvinyl)-5-methyl-6-oxo-3,6-dihydropyrimidine-4-carboxylic acid; and
- **7.44** is 2,4-di(benzo[d][1,3]dioxol-5-yl)-4-oxobutanoic acid.

The results are summarized in Table 1 here below:

**Table 1: In vitro inhibition of NHR2 tetramerization^{[a]}**

| Comp. Number | ELISA | | | ABCD | |
|---|---|---|---|---|---|
| | *IC₅₀* [µM] | Inhibition [%]^{[b]} | | Inhibition [%]^{[c]} | |
| | NHR2^{[d]} | NHR2^{[d]} | BCR^{[e]} | NHR2^{[d]} | BCR^{[e]} |
| **P1** | 460 | 42.8 | 9.2 | N.A. | N.A. |
| **P3** | » 2000 | N.A. | N.A. | N.A. | N.A. |
| **2b** | N.A. | 83.1 | 41.5 | 49.4 | 11.8 |
| **5.4** | N.A. | 89.7 | 35.4 | 63.9 | -3.3 |
| **5.8** | N.A. | 70.3 | 12.0 | 68.2 | 56.1 |
| **5.9** | N.A. | 51.7 | 20.7 | 52.5 | 14.6 |
| **5.17** | N.A. | 41.7 | 15.9 | 89.5 | 95.3 |
| **5.18** | N.A. | 80.5 | 3.9 | 83.0 | 52.6 |
| **7.3** | N.A. | 94.4 | 31.5 | 57.7 | 28.5 |
| **7.4** | N.A. | 71.6 | 10.0 | 47.4 | 6.1 |
| **7.18** | N.A. | 47.7 | -6.7 | 80.2 | -2.2 |
| **7.38** | » 2000 | 4.0 | N.A. | 20.1 | 26.1 |
| **7.44** | 680 | 69.0 | 24.0 | N.A. | N.A. |

| | | | | | |
|---|---|---|---|---|---|
| [a] Technical details of ELISA and ABCD assays are described in *Materials and Methods;* [b] relative inhibition at 1 mM inhibitor concentration. Inhibition by **2b** was measured at 50 µM concentration; inhibition by **7.3, 7.4, 7.18,** and **7.38** was measured at 2 mM concentration; [c] relative inhibition at 1 mM inhibitor concentration; [d] inhibition of tetramerization of the RUNX1-NHR2 protein; [e] inhibition of tetramerization of the RUNX1-BCR. | | | | | |

The effect of the inhibition of NHR2 tetramerization was measured in a mouse model (xenotransplantation of SKNO-1 and K562 cells in SCID mice).

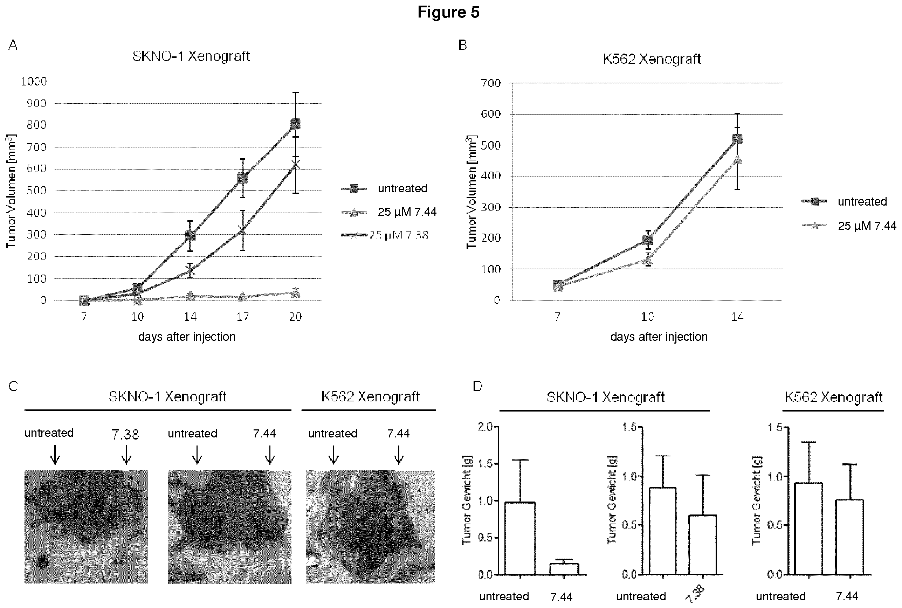

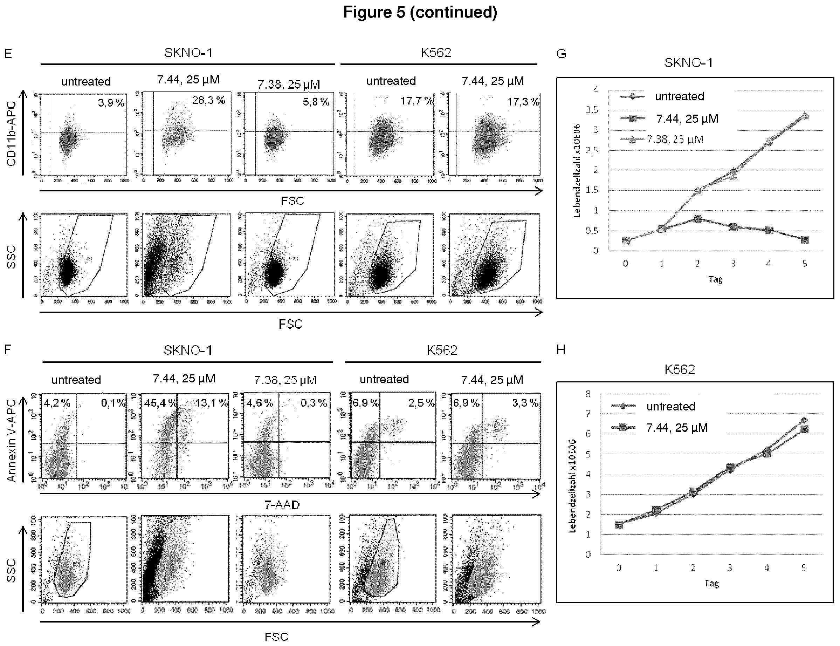

For the experiment, the SCID- or SCID-gamma chain mouse model was used. The experiment investigates if the transplantation of the AML cell line SKNO-1 after treatment with either **7.44** or **7.38** leads to reduced tumor growth. As a control, the CML cell line K562 treated with **7.44** was used. For this, the cells were treated *in vitro* with 25 µM of the respective compound for four days. Furthermore, untreated SKNO-1 and CML cell lines were used. The cell growth measured daily during this period is shown in Figure 5 g) and h). The results show that **7.44** did not influence the growth of K562 cells *in vitro* but did influence the proliferation of SKNO-1 cells *in vitro.* At day 5 the number of living cells was determined, and 5x105 SKNO-1 cells or 5x105 K562 cells were injected per transplant. Per mouse, untreated and treated cells were injected into the left and right flank, respectively.

At the day of injection, the expression of the surface marker CD11b, a measure for cell differentiation, was determined (Figure 5 e) as was the rate of apoptosis (Figure 5 f). Treatment with **7.44** led to an increase of the CD11b expression by 24% in SKNO-1 cells and an increase of the number of early apoptotic cells by 40%. In contrast, treatment of K562 cells with **7.44** did neither lead to an increase of the CD11b expression nor of the rate of apoptosis. The control **7.38** only led to an increase of 2% of CD11b expression in SKNO-1 cells. For determining the tumor volume, the length and the width of the tumor was measured twice weekly, starting one week after the transplantation (Figure 5 a and b). For SKNO-1 cells, treatment with **7.44** led to a strong reduction in tumor growth in contrast to untreated SKNO-1 cells. Treatment with **7.38** only led to a small reduction, which suggests that the solvent itself may have a small influence. Finally, treatment of K562 cells with **7.44** did not show any significant influence on tumor growth. At the end of the experiment, the tumors were freed and weighed (Figure 5c and d). The results show again that **7.44** considerably reduced tumor growth of SKNO-1 cells.

In summary, the data show a specific action of **7.44** against tumor growth of RUNX1/ETO-positive SKNO-1 cells.

Figure 5 shows reduced tumor growth of SKNO-1 cells in SCID-gc mice after treatment with **7.44.**
a) SKNO-1 cells were treated daily for 4 days with 25 µM of the compounds **7.44** or **7.38.** On day 5, 5x105 SKNO-1 cells were injected subcutaneously into the left (untreated cells) and right flank (cells treated with **7.44** or **7.38).** One week after injection, the size of the tumor was measured with a caliper.
b) K562 cells were treated daily for 4 days with 25 µM of compound **7.44.** On day 5, 5x105 K562-1 cells were injected subcutaneously into the left (untreated cells) and right flank (cells treated with **7.44**). One week after injection, the size of the tumor was measured with a caliper.
c) Freed tumors that grew after xenotransplantation of treated or untreated SKNO-1 or K562 cells.
d) Tumor weight of the xenotransplants.
e) FACS analyses of the CD11b expression of SKNO-1 cells and K562 cells before transplantation.
f) FACS analyses of annexin V- and 7-AAD-positive cells of the SKNO-1 und K562 cell lines before transplantation.
g, h) In parallel to the daily treatment with compounds, the live cell number of SKNO-1 (g) and K562 (h) cells was determined *in vitro* by trypan blue.

### Materials & Methods

### Peptides and Small Molecule Inhibitors

Peptides were synthesized by GenScript and Dr. Diana Imhof (University of Bonn). Control peptides were kindly provided by Dr. Joachim Koch (Georg-Speyer-Haus). Peptides were dissolved in dH₂O. Compounds were obtained from (Sigma Aldrich), (Enamine), (Chembridge), (TimTec), (Princeton), (Otava), (National Cancer Institute), (Chemonaut).

Compounds were dissolved in DMSO only or in dH₂O followed by dropwise addition of ammonia solution to a final concentration of 25%.

### Protein Preparation

For protein expression, chemically competent BL21(DE3) *E. coli* (Invitrogen) were used. An overnight pre-culture containing ampicillin (100 µg ml⁻¹) and glucose (0.8% w/v) was used the next day to inoculate a fresh culture at a ratio of 1:10. At an OD₆₀₀ value of 0.7, protein expression was induced by IPTG (250 µM for the NHR2 protein, 100 µM for the RUNX1-NHR2 protein, and 500 µM for the RUNX1-BCR protein), and the culture was incubated for 4 h at 37°C. For protein lysis, the bacterial pellet was resuspended in IMAC buffer (20 mM Tris-HCI, 500 mM NaCl, 10% glycerine, 20 mM imidazole, pH 8.0) in the presence of a protease inhibitor cocktail (P8849, Sigma-Aldrich). Cell lysis was performed by the addition of lysozyme (1 mg ml⁻¹) and subsequent sonication. For protein purification, the HisTrap HP column (GE Healthcare) was used.

### Cross-Linking Assay

Purified NHR2 protein (5 µM) was incubated with the peptides at 4°C for 1 h. The BS³ (Bis[sulfosuccinimidyl] suberate) crosslink-reaction was performed at a final concentration of 1 mM for 30 min at RT. Tris-HCI (0.05 M, pH 7.4) was added to the reaction mix and incubated for 10 min to stop the reaction. Protein-oligomerization was analyzed by western blotting. For protein detection, the membrane was incubated with a primary anti-myc antibody (9E10, Santa Cruz) and a secondary HRP-coupled antibody (goat anti-mouse IgG-HRP, Santa Cruz).

### ELISA Assay

Wells of a 96 well plate were coated with 100 ng streptavidin (Dianova) in PBS over night at 4°C. A double-stranded biotinylated RUNX3 oligonucleotide (100 ng, R3 = 5'-AGG GCC TGG CCT TGT GGT TCT GTG GTT GAG GGA CCA GGC-3') was bound to streptavidin in PBS containing 0.1% BSA and 0.05% Tween-20 for 2 h at RT on the 96 well plate. RUNX1-containing proteins were incubated with synthetic peptides or chemical compounds in binding buffer (25 mM HEPES, pH 7.5, 50 mM KCI, 1 mM EDTA, pH 8.0, 10 mM MgCI2, 5% glycerol, 1 mM DTT) in the presence of 1 µg salmon sperm and 1% IGEPAL CA-630 for 5 h and subsequently added to the streptavidin-bound R3 oligonucleotide for 15 min on the 96 well plate. Binding of RUNX1-containing proteins to the R3 oligonucleotide was detected with a primary anti-myc antibody (9E10, Santa Cruz) and a secondary HRP-coupled antibody (donkey anti-mouse IgG-HRP, Santa Cruz). ELISA was developed using the Sure Blue TMB Microwell Peroxidase substrate (KLP), and the reaction was stopped with sulfuric acid (1 N). Absorption at 450 nm and 650 nm was measured with the Spectra Max 340, Molecular Devices.

### ABCD Assay

The sequence of the 5'-biotinylated oligonucleotide corresponding to the RUNX1 binding sequences within RUNX3 (R3) and its mutant (R3mut) used in this study were: R3 = 5' AGG GCC TGG CCT TGT GGT TCT GTG GTT GAG GGA CCA GGC; R3mut = 5' AGG GCC TGG CCT TGT TAG TCT GTT AGT GAG GGA CCA GGC. Oligonucleotides were annealed to the corresponding unmodified antisense oligonucleotide to generate the RUNX1 binding site. Purified RUNX1 proteins were pre-incubated with a compound for 1 h at 4°C. Similarly the biotinylated double-stranded oligonucleotides were pre-incubated with streptavidin coated magnetic beads (Dynabeads M-280 Streptavidin, Invitrogen) for 1 h at 4°C. Protein/compound samples and beads with bound oligonucleotide were incubated in binding buffer (25 mM HEPES, pH 7.5, 50 mM KCI, 1 mM EDTA, pH 8.0, 10 mM MgCl₂, 5% glycerol, 1 mM DTT) in the presence of 2% IGEPAL CA-630, 1 µg of salmon sperm, and 0.1% BSA for 1½ h. After washing three times, the bound proteins were eluted from beads in SDS-PAGE sample buffer and resolved on SDS-PAGE followed by immunoblotting with a primary anti-myc (9E10, Santa Cruz) antibody and a secondary HRP-coupled antibody (goat anti-mouse IgG-HRP, Santa Cruz).

### Cell Survival Assays

SKNO-1 cells were cultured in RPMI 1640 + 10% FCS and 7 ng ml⁻¹ human GM-CSF. U937 cells were cultured in RPMI 1640 + 10% FCS. Cell proliferation and viability were measured with the cell proliferation kit II (XTT, Roche Applied Science). 3000 cells per well were cultured in a 96 well plate and daily treated with compounds at different concentrations. The XTT assay was performed at days 3, 5, and 7.

## Claims

1. A compound according to general formula (A), 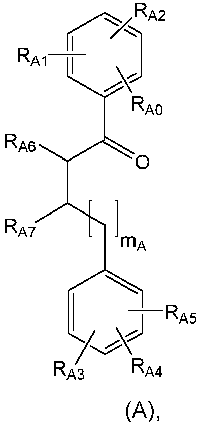 wherein
R_{A0}, R_{A1} and R_{A2}, respectively independently, mean -H, -F or -OC₁₋₈-aliphatic;
or R_{A0} means -H, -F or -OC₁₋₈-aliphatic; and R_{A1} and R_{A2} are vicinal and together with the carbon atoms to which they are attached form a ring and mean -O-CH₂-O-or -O-CH(C₁₋₈-aliphatic)-O-;
R_{A3}, R_{A4}, and R_{A5}, respectively independently, mean -H, -F or -OC₁₋₈-aliphatic;
or R_{A3} means -H, -F or -OC₁₋₈-aliphatic; and R_{A4} and R_{A5} are vicinal and together with the carbon atoms to which they are attached form a ring and mean -O-CH₂-O-or -O-CH(C₁₋₈-aliphatic)-O-;
R_{A6} means -H or -C₁₋₈-aliphatic;
R_{A7} means -C(=O)OH, -C(=O)OR_{X}, -C(=O)NH₂, -C(=O)NHR_{X}, or -C(=O)N(R_{X})₂; and
m_{A} means 0, 1, 2, 3, or 4;
or a physiologically acceptable salt thereof;
wherein Rx, respectively independently, means -C₁₋₈-aliphatic, -C₃₋₁₂-cycloaliphatic, -aryl, -heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁₋₈-aliphatic, -C₃₋₈-cycloaliphatic-aryl or -C₃₋₈-cycloaliphatic-heteroaryl;
wherein "aliphatic", respectively independently, means a branched or unbranched, saturated or a mono- or polyunsaturated, unsubstituted or mono- or polysubstituted, aliphatic hydrocarbon residue;
wherein "cycloaliphatic", respectively independently, means a saturated or a mono- or polyunsaturated, unsubstituted or mono- or polysubstituted, alicyclic, mono- or multicyclic hydrocarbon residue;
wherein with respect to "aliphatic" and "cycloaliphatic", "mono- or polysubstituted", respectively independently, means the mono- or polysubstitution of one or more hydrogen atoms by -F, -CI, -Br, -I, -CN, -NO₂, -CHO, =O, -Rx, -C(=O)R_{X}, -C(=O)H, -C(=O)OH, -C(=O)OR_{X}, -C(=O)NH₂, -C(=O)NHR_{X}, -C(=O)N(R_{X})₂, -OH, -ORx, -OC(=O)H, -OC(=O)R_{X}, -OC(=O)-OR_{X}, -OC(=O)NHR_{X}, -OC(=O)N(R_{X})₂, -SH, -SRx, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NHC(=O)NH₂, -NHC(=O)NHR_{X}, -NHC(=O)-N(R_{X})₂, -Si(R_{X})₃ or -PO(OR_{X})₂;
wherein "aryl", respectively independently, means a carbocyclic ring system with at least one aromatic ring, but without heteroatoms in this ring, wherein, if necessary, the aryl residues can be condensed with further saturated, (partially) unsaturated or aromatic ring systems, and each aryl residue can be present in unsubstituted or mono- or polysubstituted form, wherein the aryl substituents can be the same or different and in any desired and possible position of the aryl;
wherein "heteroaryl", respectively independently, means a 5-, 6- or 7-membered cyclic aromatic residue, which contains 1, 2, 3, 4 or 5 heteroatoms, wherein the heteroatoms, the same or different, are nitrogen, oxygen or sulphur, and the heterocycle can be unsubstituted or mono- or polysubstituted; wherein in the case of the substitution on the heterocycle the substituents can be the same or different and can be in any desired and possible position of the heteroaryl; and wherein the heterocycle can also be part of a bi- or polycyclic system;
wherein with respect to "aryl" and "heteroaryl", "mono- or polysubstituted", respectively independently, means the mono- or polysubstitution of one or more hydrogen atoms of the ring system by substituents selected from the group comprising -F, -CI, -Br, -I, -CN, -NO₂, -CHO, -R_{X}, -C(=O)R_{X}, -C(=O)H, -C(=O)OH, -C(=O)OR_{X}, -C(=O)NH₂, - C(=O)NHR_{X}, -C(=O)-N(R_{X})₂, -OH, -O(CH₂)₁₋₂O-, -ORx, -OC(=O)H, -OC(=O)R_{X}, -OC(=O)OR_{X}, -OC(=O)NHR_{X}, -OC(=O)N(R_{X})₂, -SH, -SRx, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NH-C(=O)NH₂, -NHC(=O)NHR_{X}, -NHC(=O)-N(R_{X})₂, -Si(R_{X})₃ and -PO(OR_{X})₂; wherein if necessary N-ring atoms present can be respectively oxidized;
for use in the treatment or the prevention of leukemia.

2. The compound for use according to claim 1,
wherein
R_{A0} means -H;
R_{A1} and R_{A2}, respectively independently, mean -OC₁₋₈-alkyl;
or R_{A1} and R_{A2} are vicinal and together with the carbon atoms to which they are attached form a ring and mean -O-CH₂-O- or -O-CH(C₁₋₈-alkyl)-O-;
R_{A3}, R_{A4}, and R_{A5}, respectively independently, mean -H or -OC₁₋₈-alkyl;
or R_{A3} means -H or -OC₁₋₈-alkyl; and R_{A4} and R_{A5} are vicinal and together with the carbon atoms to which they are attached form a ring and mean -O-CH₂-O- or -O-CH(C₁₋₈-alkyl)-O-;
R_{A6} means -H or -C₁₋₈-alkyl;
R_{A7} means -C(=O)OH or -C(=O)OR_{X}; and
m_{A} means 0, 1, or 2.

3. The compound for use according to claim 1 or 2,
wherein
R_{A0} means -H;
R_{A1} and R_{A2}, respectively independently, mean -OCH₃;
or R_{A1} and R_{A2} are vicinal and together with the carbon atoms to which they are attached form a ring and mean -O-CH₂-O- or -O-CH(CH₃)-O-;
R_{A3}, R_{A4}, and R_{A5}, respectively independently, mean -H or -OCH₃;
or R_{A3} means -H; and R_{A4} and R_{A5} are vicinal and together with the carbon atoms to which they are attached form a ring and mean -O-CH₂-O- or -O-CH(CH₃)-O-;
R_{A6} means -H or -CH₃;
R_{A7} means -CO₂H; and
m_{A} means 0 or 1.

4. The compound for use according to any of the preceding claims, which is selected from the group consisting of
- 2,4-di(benzo[d][1,3]dioxol-5-yl)-4-oxobutanoic acid;
- 2-(benzo[d][1,3]dioxol-5-yl)-4-(2-methylbenzo[d][1,3]dioxol-5-yl)-4-oxobutanoic acid;
- 4-(benzo[d][1,3]dioxol-5-yl)-3-methyl-4-oxo-2-(3,4,5-trimethoxybenzyl)butanoic acid;
and the physiologically acceptable salts thereof.

5. The compound for use according to any of the preceding claims, wherein the leukemia is myeloid leukemia.

6. The compound for use according to claim 5, wherein the myeloid leukemia is selected from the group consisting of acute myeloid leukemia, promyeloid leukemia, acute promyeloid leukemia, promyelocytic leukemia, acute promyelocytic leukemia, megakaryoblastic leukemia and acute megakaryoblastic leukemia.

7. The compound for use according to any of the preceding claims, which is administered once daily, twice daily, thrice daily or more often to a subject in need thereof or continuously infused.

8. The compound for use according to any of the preceding claims, which is administered (i) systemically, locally or extracorporeally; and/or (ii) orally or parenterally.

## Patentansprüche

1. Eine Verbindung gemäß der allgemeinen Formel (A), 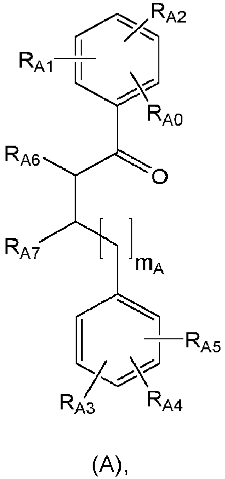 wobei
R_{A0}, R_{A1} und R_{A2} jeweils unabhängig voneinander -H, -F oder -OC₁₋₈-Aliphatisch bedeuten;
oder R_{A0} -H, -F oder -OC₁₋₈-Aliphatisch bedeutet; und R_{A1} und R_{A2} vicinal sind und zusammen mit den Kohlenstoffatomen, an die sie gebunden sind, einen Ring bilden und -O-CH₂-O- oder -O-CH(C₁₋₈-Aliphatisch)-O- bedeuten;
R_{A3}, R_{A4} und R_{A5} jeweils unabhängig voneinander -H, -F oder -OC₁₋₈-Aliphatisch bedeuten;
oder R_{A3} -H, -F oder -OC₁₋₈-Aliphatisch bedeutet; und R_{A4} und R_{A5} vicinal sind und zusammen mit den Kohlenstoffatomen, an die sie gebunden sind, einen Ring bilden und -O-CH₂-O- oder -O-CH(C₁₋₈-Aliphatisch)-O- bedeuten;
R_{A6} -H oder -C₁₋₈-Aliphatisch bedeutet;
R_{A7} -C(=O)OH, -C(=O)OR_{X}, -C(=O)NH₂, -C(=O)NHR_{X} oder -C(=O)N(R_{X})₂ bedeutet; und
m_{A} 0, 1, 2, 3 oder 4 bedeutet;
oder deren physiologisch verträgliches Salz;
wobei Rx, jeweils unabhängig voneinander, -C₁₋₈-Aliphatisch, -C₃₋₁₂-Cycloaliphatisch, -Aryl, - Heteroaryl, -C₁₋₈-Aliphatisch-C₃₋₁₂-Cycloaliphatisch, -C₁₋₈-Aliphatisch-Aryl, -C₁₋₈-Aliphatisch-Heteroaryl, -C₃₋₈-Cycloaliphatisch-C₁₋₈-Aliphatisch, -C3-8-Cycloaliphatisch-Aryl oder -C₃₋₈-Cycloaliphatisch-Heteroaryl bedeutet;
wobei "Aliphatisch" jeweils unabhängig einen verzweigten oder unverzweigten, gesättigten oder einen einfach oder mehrfach ungesättigten, unsubstituierten oder einfach oder mehrfach substituierten, aliphatischen Kohlenwasserstoffrest bedeutet;
wobei "Cycloaliphatisch" jeweils unabhängig einen gesättigten oder einen einfach oder mehrfach ungesättigten, unsubstituierten oder einfach oder mehrfach substituierten, alizyklischen, mono- oder multizyklischen Kohlenwasserstoffrest bedeutet;
wobei in Bezug auf "Aliphatisch" und "Cycloaliphatisch", "einfach oder mehrfach substituiert", jeweils unabhängig die Einfach- oder Mehrfachsubstitution eines oder mehrerer Wasserstoffatome durch -F, -Cl, -Br, -I, -CN, -NO₂, -CHO, =O, -Rx, -C(=O)R_{X}, -C(=O)H, - C(=O)OH, -C(=O)OR_{X}, -C(=O)NH₂, -C(=O)NHR_{X}, -C(=O)N(R_{X})₂, -OH, -ORx, -OC(=O)H, - OC(=O)R_{X}, -OC(=O)-OR_{X}, -OC(=O)NHR_{X}, -OC(=O)N(R_{X})₂, -SH, -SRx, -SO₃H, -S(=O)₁₋₂-R_{X}, - S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, - NHC(=O)NH₂, -NHC(=O)NHR_{X}, -NHC(=O)-N(R_{X})₂, -Si(R_{X})₃ oder -PO(OR_{X})₂ bedeutet;
wobei "Aryl" jeweils unabhängig ein carbozyklisches Ringsystem mit mindestens einem aromatischen Ring, jedoch ohne Heteroatome in diesem Ring, bedeutet, wobei die Arylreste, sofern notwendig, mit weiteren gesättigten, teilweise ungesättigten, ungesättigten oder aromatischen Ringsystemen kondensiert sein können und jeder Arylrest in unsubstituierter oder einfach oder mehrfach substituierter Form vorliegen kann, wobei die Arylsubstituenten gleich oder verschieden und in jeder gewünschten und möglichen Position des Aryls sein können;
wobei "Heteroaryl" jeweils unabhängig einen 5-, 6- oder 7-gliedrigen zyklischen aromatischen Rest bedeutet, der 1, 2, 3, 4 oder 5 Heteroatome enthält, wobei die Heteroatome, gleich oder verschieden, Stickstoff, Sauerstoff oder Schwefel sind und der Heterozyklus unsubstituiert oder einfach oder mehrfach substituiert sein kann; wobei im Falle der Substitution am Heterozyklus die Substituenten gleich oder verschieden sein können und in jeder gewünschten und möglichen Position des Heteroaryls sein können; und wobei der Heterozyklus auch Teil eines bizyklischen oder polyzyklischen Systems sein kann;
wobei in Bezug auf "Aryl" und "Heteroaryl", "einfach oder mehrfach substituiert" jeweils unabhängig die Einfach- oder Mehrfachsubstitution eines oder mehrerer Wasserstoffatome des Ringsystems durch Substituenten ausgewählt aus der Gruppe umfassend -F, -Cl, -Br, -I, - CN, -NO₂, -CHO, -R_{X}, -C(=O)R_{X}, -C(=O)H, -C(=O)OH, -C(=O)OR_{X}, -C(=O)NH₂, -C(=O)NHR_{X}, -C(=O)-N(R_{X})₂, -OH, -O(CH₂)₁₋₂O-, -ORx, -OC(=O)H, -OC(=O)R_{X}, -OC(=O)OR_{X}, - OC(=O)NHR_{X}, -OC(=O)N(R_{X})₂, -SH, -SR_{X}, -SO₃H, -S(=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, -NHC(=O)R_{X}, -NHC(=O)OR_{X}, -NH-C(=O)NH₂, -NHC(=O)NHR_{X}, - NHC(=O)-N(R_{X})₂, -Si(R_{X})₃ und -PO(OR_{X})₂ bedeutet; wobei, sofern notwendig, vorhandene N-Ringatome jeweils oxidiert sein können;
zur Verwendung bei der Behandlung oder der Prävention von Leukämie.

2. Die Verbindung zur Verwendung gemäß Anspruch 1,
wobei
R_{A0} -H bedeutet;
R_{A1} und R_{A2} jeweils unabhängig voneinander -OC₁₋₈-Alkyl bedeuten;
oder R_{A1} und R_{A2} vicinal sind und zusammen mit den Kohlenwasserstoffatomen, an die sie gebunden sind, einen Ring bilden und -O-CH₂-O- oder -O-CH(C₁₋₈-Alkyl)-O-bedeuten;
R_{A3}, R_{A4} und R_{A5} jeweils unabhängig voneinander -H oder -OC₁₋₈-Alkyl bedeuten; oder R_{A3} -H oder -OC₁₋₈-Alkyl bedeutet; und R_{A4} und R_{A5} vicinal sind und zusammen mit den Kohlenwasserstoffatomen, an die sie gebunden sind, einen Ring bilden und -O-CH₂-O- oder -O-CH(C₁₋₈-Alkyl)-O- bedeuten;
R_{A6} -H oder -C₁₋₈-Alkyl bedeutet;
R_{A7} -C(=O)OH oder -C(=O)OR_{X} bedeutet; und
m_{A} 0, 1 oder 2 bedeutet.

3. Die Verbindung zur Verwendung gemäß Anspruch 1 oder 2,
wobei
R_{A0} -H bedeutet;
R_{A1} und R_{A2} jeweils unabhängig voneinander -OCH₃ bedeuten;
oder R_{A1} und R_{A2} vicinal sind und zusammen mit den Kohlenwasserstoffatomen, an die sie gebunden sind, einen Ring bilden und -O-CH₂-O- oder -O-CH(CH₃)-O- bedeuten;
R_{A3}, R_{A4} und R_{A5} jeweils unabhängig voneinander -H oder -OCH₃ bedeuten;
oder R_{A3} -H bedeutet; und R_{A4} und R_{A5} vicinal sind und zusammen mit den Kohlenwasserstoffatomen, an die sie gebunden sind, einen Ring bilden und -O-CH₂-O-oder -O-CH(CH₃)-O- bedeuten;
R_{A6} -H oder -CH₃ bedeutet;
R_{A7} -CO₂H bedeutet; und
m_{A} 0 oder 1 bedeutet.

4. Die Verbindung zur Verwendung gemäß einem der vorhergehenden Ansprüche, die ausgewählt wird aus der Gruppe bestehend aus
- 2,4-Di(benzo[d][1,3]dioxol-5-yl)-4-oxobutansäure;
- 2-(Benzo[d][1,3]dioxol-5-yl)-4-(2-methylbenzo[d][1,3]dioxol-5-yl)-4-oxobutansäure;
- 4-(Benzo[d][1,3]dioxol-5-yl)-3-methyl-4-oxo-2-(3,4,5-trimethoxybenzyl)butansäure;
und deren physiologisch verträgliche Salze.

5. Die Verbindung zur Verwendung gemäß einem der vorhergehenden Ansprüche, wobei die Leukämie eine myeloische Leukämie ist.

6. Die Verbindung zur Verwendung gemäß Anspruch 5, wobei die myeloische Leukämie ausgewählt wird aus der Gruppe bestehend aus akuter myeloischer Leukämie, promyeloischer Leukämie, akuter promyeloischer Leukämie, Promyelozytenleukämie, akuter Promyelozytenleukämie, megakaryoblastischer Leukämie und akuter megakaryoblastischer Leukämie.

7. Die Verbindung zur Verwendung gemäß einem der vorhergehenden Ansprüche, die einmal täglich, zweimal täglich, dreimal täglich oder öfter an ein Subjekt, das diese benötigt, verabreicht oder kontinuierlich infundiert wird.

8. Die Verbindung zur Verwendung gemäß einem der vorhergehenden Ansprüche, die (i) systemisch, lokal oder extrakorporal; und/oder (ii) oral oder parenteral verabreicht wird.

## Revendications

1. Composé selon la formule générale (A), 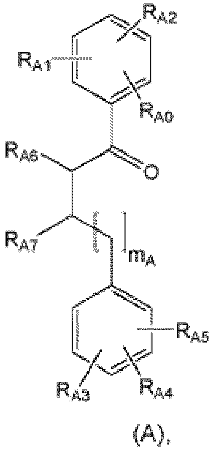
R_{A0}, R_{A1} et R_{A2}, respectivement indépendamment, signifiant -H, -F ou -OC₁₋₈-aliphatique ;
ou R_{A0} signifiant -H, -F ou -OC₁₋₈-aliphatique ; et R_{A1} et R_{A2} étant vicinaux et conjointement avec les atomes de carbone auxquels ils sont fixés formant un cycle et signifiant -O-CH₂-O- ou -O-CH(C₁₋₈-aliphatique)-O-;
R_{A3}, R_{A4}, et R_{A5}, respectivement indépendamment, signifiant -H, -F ou -OC₁₋₈-aliphatique;
ou R_{A3} signifiant -H, -F ou -OC₁₋₈-aliphatique ; et R_{A4} et R_{A5} étant vicinaux et conjointement avec les atomes de carbone auxquels ils sont fixés formant un cycle et signifiant -O-CH₂-O- ou -O-CH(C₁₋₈-aliphatique)-O-;
R_{A6} signifiant -H ou -C₁₋₈-aliphatique ;
R_{A7} signifiant -C(=O)OH, -C(=O)OR_{X}, -C(=O)NH₂, - C(=O)NHR_{X}, ou -C (=O)N(R_{X})₂; et
m_{A} signifiant 0, 1 , 2, 3, ou 4 ;
ou sel physiologiquement acceptable correspondant ;
R_{X}, respectivement indépendamment, signifiant -C₁₋₈-aliphatique, -C₃₋₁₂-cycloaliphatique, -aryle,-hétéroaryle, -C₁₋₈-aliphatique-C₃₋₁₂-cycloaliphatique, -C₁₋₈-aliphatique-aryle, -C₁₋₈-aliphatique-hétéroaryle, -C₃₋₈-cycloaliphatique-C₁₋₈-aliphatique, -C₃₋₈-cycloaliphatique-aryle ou -C₃₋₈-cycloaliphatique-hétéroaryle ;
« aliphatique », respectivement indépendamment, signifiant un radical hydrocarboné aliphatique ramifié ou non ramifié, saturé ou monoinsaturé ou polyinsaturé, non substitué ou monosubstitué ou polysubstitué ;
« cycloaliphatique », respectivement indépendamment, signifiant un radical hydrocarboné saturé ou monoinsaturé ou polyinsaturé, non substitué ou monosubstitué ou polysubstitué, alicyclique, monocyclique ou polycyclique ;
par rapport à « aliphatique » et « cycloaliphatique », « monosubstitué ou polysubstitué », respectivement indépendamment, signifiant la monosubstitution ou la polysubstitution d'un ou plusieurs atomes d'hydrogène par -F, -Cl, -Br, -I, -CN, -NO₂, -CHO, =O, -R_{X}, -C(=O)R_{X}, -C(=O)H, -C(=O)OH, -C(=O)OR_{X}, - C(=O)NH₂, -C(=O)NHR_{X}, -C(=O)N(R_{X})₂, -OH, -OR_{X}, - OC(=O)H, -OC(=O)R_{X}, -OC(=O)-OR_{X}, -OC(=O)NHR_{X}, - OC(=O)N(R_{X})₂, -SH, -SR_{X}, -SO₃H, -S (=O)₁₋₂-R_{X}, -S(=O)₁₋₂NH₂, -NH₂, -NHR_{X}, -N(R_{X})₂, -N⁺(R_{X})₃, -N⁺(R_{X})₂O⁻, - NHC(=O)R_{X}, -NHC (=O)OR_{X}, -NHC(=O)NH₂, -NHC (=O)NHR_{X}, - NHC(=O)-N(R_{X})₂, -Si(R_{X})₃ ou -PO(OR_{X})₂;
« aryle », respectivement indépendamment, signifiant un système cyclique carbocyclique comportant au moins un cycle aromatique, mais sans hétéroatomes dans ce cycle, si nécessaire, les radicaux aryles pouvant être condensés avec d'autres systèmes cycliques saturés, (partiellement) insaturés ou aromatiques, et chaque radical aryle pouvant être présent sous forme non substituée ou monosubstituée ou polysubstituée, les substituants d'aryle pouvant être identiques ou différents et en une quelconque position souhaitée et possible sur l'aryle;
« hétéroaryle », respectivement indépendamment, signifiant un radical aromatique cyclique à 5, 6 ou 7 chaînons, qui contient 1, 2, 3, 4 ou 5 hétéroatomes, les hétéroatome, identiques ou différents, étant azote, oxygène ou soufre, et l'hétérocycle pouvant être non substitué ou monosubstitué ou polysubstitué ; dans le cas de la substitution sur l'hétérocycle, les substituants pouvant être identiques ou différents et pouvant être en une quelconque position souhaitée et possible sur l'hétéroaryle ; et l'hétérocycle pouvant également faire partie d'un système bicyclique ou polycyclique ;
par rapport à « aryle » et « hétéroaryle », « monosubstitué ou polysubstitué », respectivement indépendamment, signifiant la monosubstitution ou la polysubstitution d'un ou plusieurs atomes d'hydrogène du système cyclique par des substituants choisis dans le groupe comprenant -F, -Cl, -Br, -I, -CN, -NO₂, -CHO, -R_{X}, -C(=O)R_{X}, - C(=O)H, -C(=O)OH, -C(=O)OR_{X}, -C(=O)NH₂, -C(=O)NHR_{X}, -C(=O)-N(R_{X})₂, -OH, -O(CH₂)₁₋₂O-, -OR_{X}, -OC(=O)H, - OC (=O)R_{X}, -OC(=O)OR_{X}, -OC(=O)NHR_{X}, -OC(=O)N(R_{X})₂, - SH, -SR_{X}, -SO₃H, -S(=O)₁₋₂-R_{X}, -S(=O)₁₋₂NH₂, -NH₂, - NHR_{X}, -N(R_{X})₂, -N⁺(R_{X})₃, -N⁺(R_{X})₂O⁻, -NHC(=O)R_{X}, - NHC(=O)OR_{X}, -NH-C(=O)NH₂, -NHC(=O)NHR_{X}, -NHC(=O)-N(R_{X})₂, -Si(R_{X})₃ et -PO(OR_{X})₂; si nécessaire des atomes N de cycle présents pouvant être respectivement oxydés ;
pour une utilisation dans le traitement ou la prévention d'une leucémie.

2. Composé pour une utilisation selon la revendication 1,
R_{A0} signifiant -H ;
R_{A1} et R_{A2}, respectivement indépendamment, signifiant -OC₁₋₈-alkyle ;
ou R_{A1} et R_{A2} étant vicinaux et conjointement avec les atomes de carbone auxquels ils sont fixés formant un cycle et signifiant -O-CH₂-O- ou -O-CH(C₁₋₈-alkyle)-O-;
R_{A3}, R_{A4}, et R_{A5}, respectivement indépendamment, signifiant -H ou -OC₁₋₈-alkyle;
ou R_{A3} signifiant -H ou -OC₁₋₈-alkyle ; et R_{A4} et R_{A5} étant vicinaux et conjointement avec les atomes de carbone auxquels ils sont fixés formant un cycle et signifiant -O-CH₂-O- ou -O-CH (C₁₋₈-alkyle)-O- ;
R_{A6} signifiant -H ou -C₁₋₈-alkyle;
R_{A7} signifiant -C(=O)OH ou -C(=O)OR_{X}; et
m_{A} signifiant 0, 1, ou 2.

3. Composé pour une utilisation selon la revendication 1 ou 2,
R_{A0} signifiant -H ;
R_{A1} et R_{A2}, respectivement indépendamment, signifiant -OCH₃ ;
ou R_{A1} et R_{A2} étant vicinaux et conjointement avec les atomes de carbone auxquels ils sont fixés formant un cycle et signifiant -O-CH₂-O- ou -O-CH(CH₃)-O-;
R_{A3}, R_{A4}, et R_{A5}, respectivement indépendamment, signifiant -H ou -OCH₃ ;
ou R_{A3} signifiant -H ; et R_{A4} et R_{A5} étant vicinaux et conjointement avec les atomes de carbone auxquels ils sont fixés formant un cycle et signifiant -O-CH₂-O- ou -O-CH(CH₃)-O-;
R_{A6} signifiant -H ou -CH₃;
R_{A7} signifiant -CO₂H; et
m_{A} signifiant 0 ou 1.

4. Composé pour une utilisation selon l'une quelconque des revendications précédentes, qui est choisi dans le groupe constitué par
- l'acide 2,4-di(benzo[d][1,3]dioxol-5-yl)-4-oxobutanoïque ;
- l'acide 2-(benzo[d][1,3]dioxol-5-yl)-4-(2-méthylbenzo[d][1,3]dioxol-5-yl)-4-oxobutanoïque ;
- l'acide 4-(benzo[d][1,3]dioxol-5-yl)-3-méthyl-4-oxo-2-(3,4,5-triméthoxybenzyl)butanoïque ;
et les sels physiologiquement acceptables correspondants.

5. Composé pour une utilisation selon l'une quelconque des revendications précédentes, la leucémie étant une leucémie myéloïde.

6. Composé pour une utilisation selon la revendication 5, la leucémie myéloïde étant choisie dans le groupe constitué par une leucémie myéloïde aiguë, une leucémie promyéloïde, une leucémie promyéloïde aiguë, une leucémie promyélocitaire, une leucémie promyélocitaire aiguë, une leucémie mégacaryoblastique et une leucémie mégacaryoblastique aiguë.

7. Composé pour une utilisation selon l'une quelconque des revendications précédentes, qui est administré une fois par jour, deux fois par jour, trois fois par jour ou plus souvent à un sujet qui en a besoin ou est perfusé de manière continue.

8. Composé pour une utilisation selon l'une quelconque des revendications précédentes, qui est administré (i) de manière systémique, de manière locale ou de manière extracorporelle ; et/ou (ii) de manière orale ou de manière parentérale.
